(19) 

Europäisches
Patentamt
European
Patent Office
Office européen
des brevets

(11)     **EP 4 029 983 A1**

(12)                     **EUROPEAN PATENT APPLICATION**
                    published in accordance with Art. 153(4) EPC

(43) Date of publication:
    **20.07.2022 Bulletin 2022/29**

(21) Application number: **20862987.3**

(22) Date of filing: **10.09.2020**

(51) International Patent Classification (IPC):
    *D06M 101/06* (2006.01)        *A61Q 1/00* (2006.01)
    *D21H 11/20* (2006.01)        *A61K 8/73* (2006.01)
    *C08B 5/14* (2006.01)        *D06M 13/256* (2006.01)

(52) Cooperative Patent Classification (CPC):
    **A61K 8/73; A61Q 1/00; C08B 5/14; D06M 13/256;
    D21H 11/20**

(86) International application number:
    **PCT/JP2020/034293**

(87) International publication number:
    **WO 2021/049571 (18.03.2021 Gazette 2021/11)**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **KH MA MD TN**

(30) Priority: **11.09.2019 JP 2019165599
            09.09.2020 JP 2020151595**

(71) Applicant: **Marusumi Paper Co., Ltd.
    Shikokuchuo-shi, Ehime, 799-0196 (JP)**

(72) Inventors:
    • **HIASA, Shou
      Shikokuchuo-shi, Ehime 799-0196 (JP)**
    • **NISHIYAMA, Seiji
      Shikokuchuo-shi, Ehime 799-0196 (JP)**

(74) Representative: **Plougmann Vingtoft a/s
    Strandvejen 70
    2900 Hellerup (DK)**

(54)     **SULFONATED CELLULOSE MICROFIBERS AND METHOD FOR PRODUCING SAME**

(57)     [Object] The present invention provides a sulfonated fine cellulose fiber having excellent viscosity and the like, as well as a sulfonated fine cellulose fiber production method capable of efficiently producing the sulfonated fine cellulose fibers.

[Method for Achieving the Object] A fine cellulose fiber obtained by fibrillating a cellulose fiber wherein a part of the hydroxyl groups of the fine cellulose fiber is substituted with sulfo groups, and an introduction amount of sulfur attributable to the sulfo groups is higher than 0.42 mmol/g. The fine cellulose fiber comprises a plurality of unit fibers and has an average fiber width of 30 nm or less. A dispersion liquid in which the fine cellulose fiber is dispersed in a water-soluble solvent at a solid concentration of 0.5% by mass has a viscosity of 5,000 mPa·s or more at 25°C. This makes it possible to obtain the desired viscosity when the fine cellulose fibers are dispersed in a dispersion liquid.

FIG.1

```
            PULP
             │
             ▼
POLYMERIZATION DEGREE
LOWERING TREATMENT
STEP (S1)
             │
             ▼
CHEMICAL TREATMENT STEP
(S2)
             │
             ▼
SULFONATED PULP FIBERS
             │
             ▼
FIBRILLATION TREATMENT STEP
(S3)
             │
             ▼
SULFONATED FINE CELLULOSE
FIBERS
```

EP 4 029 983 A1

**Description**

Technical Field

The present invention relates to sulfonated fine cellulose fibers and methods for producing the same.

Background Art

Cellulose nanofibers (CNF) are expected to exhibit superior thickening effects (high viscosity and thixotropy) compared to cellulose fibers, and are therefore attracting attention as a novel thickener with less environmental load.

To obtain CNF with high viscosity, it is generally considered necessary to increase the fiber length of CNF. Therefore, some technologies to prevent decreases in fiber length of pulp during chemical treatments and during fibrillation to thereby produce CNF with high viscosity have been proposed (e.g., Patent Literatures 1 and 2).

Patent Literature 1 discloses a technology relating to phosphorylated CNF, and teaches a method of increasing the polymerization degree of phosphorylated CNF to 400 or higher so that a slurry in which the phosphorylated CNFs are dispersed has a viscosity of 10,000 to 20,000 mPa•s.

However, the technology of Patent Literature 1 has a problem of significantly low production efficiency and impracticality, as it requires very complicated multiple processes to prevent decreases in fiber length during the chemical treatment steps.

If focusing only on the aspect of improving the productivity of CNF, it can be achieved by processing short fiber pulp with a high fibrillation pressure. However, in this case, the resulting CNF has a shorter fiber length, and is not likely to ensure desired viscosity. On the other hand, if the fibrillation treatment is performed slowly while suppressing the fibrillation pressure during the fibrillation, the decrease in fiber length during the fibrillation can be prevented to some extent, thereby producing CNF with fibers of a certain length. However, this method poses a problem of significantly low productivity, and therefore is impractical.

Patent Literature 2 proposes a technology that can solve the problems described above.

The technology of Patent Literature 2 relates to sulfated CNF production in which fibrillation is performed with a fibrillation pressure of 150 MPa, thereby producing sulfated CNF with a polymerization degree of 350 or higher while maintaining desired productivity. Patent Literature 2 further descloses that the resulting sulfated CNF has a viscosity of 5,400 to 8,000 mPa•s.

Citation List

Patent Literature

   Patent Literature 1: International Publication WO2017/170908
   Patent Literature 2: Japanese Laid-Open Patent Application Publication No. 2019-11411

Summary of Invention

Technical Problem

However, although the technology of Patent Literature 2 enables practical production of CNF with a certain degree of viscosity (up to 8,100 mPa•s), it is still incapable of producing CNF with high viscosity satisfying the current market need (e.g., 10,000 mPa•s or more); moreover, the technology of Patent Literature 2 requires a fibrillation treatment with high energy, i.e., 150 MPa or more. Furthermore, Patent Literature 2 nowhere teaches biodegradability or problems caused by coloring.

In view of the above circumstances, an object of the present invention is to provide sulfonated fine cellulose fibers with characteristics including excellent viscosity, as well as a sulfonated fine cellulose fiber production method capable of efficiently producing such sulfonated fine cellulose fibers. Solution to Problem

Sulfonated Fine Cellulose Fiber

The sulfonated fine cellulose fiber of the first invention is a sulfonated fine cellulose fiber, which is a fine cellulose fiber prepared by fibrillating a cellulose fiber, a part of hydroxyl groups of the fine cellulose fiber being substituted with sulfo groups, an introduction amount of sulfur attributable to the sulfo groups being adjusted to be higher than 0.42 mmol/g, the fine cellulose fiber comprising a plurality of unit fibers and having an average fiber width of 30 nm or less, and a dispersion liquid in which the fine cellulose fiber is dispersed in a water-soluble solvent at a solid concentration of 0.5% by mass having a viscosity of 5,000 mPa•s or more at 25°C.

The sulfonated fine cellulose fiber of the second invention is, in the first invention, a thixotropy index determined by measuring the dispersion liquid in which the fine cellulose fiber is dispersed in a water-soluble solvent at a solid concentration of 0.5% by mass using a Brookfield viscometer at rotation speeds of 6 rpm and 60 rpm at 25°C, calculating each viscosity, and determining each viscosity ratio (viscosity at a rotation speed of 6 rpm/viscosity at a rotation speed of 60 rpm) is 5 or more.

The sulfonated fine cellulose fiber of the third invention is, in the first invention, a thixotropy index determined by measuring the dispersion liquid in which the fine cellulose fiber is dispersed in a water-soluble solvent at a solid concentration of 0.5% by mass using a Brookfield viscometer at rotation speeds of 6 rpm and 60 rpm at 25°C, calculating each viscosity, and determining each viscosity ratio (viscosity at a rotation speed of 6 rpm/viscosity at a rotation speed of 60 rpm) is less than 5.0.

The sulfonated fine cellulose fiber of the fourth invention is, in the first, second or third invention, the unit fibers have an average polymerization degree of 350 or less.

The sulfonated fine cellulose fiber of the fifth invention is, in the first, second, third, or fourth invention, the dispersion liquid in which the fine cellulose fiber is dispersed in a water-soluble solvent at a solid concentration of 0.5% by mass has a haze value of 10% or less.

The sulfonated fine cellulose fiber of the sixth invention is, in the first, second, third, fourth, or fifth invention, the dispersion liquid in which the fine cellulose fiber is dispersed in a water-soluble solvent at a solid concentration of 0.5% by mass has a total light transmittance of 90% or more.

The sulfonated fine cellulose fiber of the seventh invention is, in any one of the first to the sixth invention, the L*a*b* color space, which is determined according to JIS Z 8781-4:2013, of the dispersion liquid in which the fine cellulose fiber is dispersed in a water-soluble solvent at a solid concentration of 0.5% by mass satisfies at least one of the following conditions of L*, a* and b*:

L* is 95% or more;
a* is not less than -5 and not more than +5; and
b* is not less than -5 and not more than +5.

Method for Producing Sulfonated Fine Cellulose Fibers

The method for producing a sulfonated fine cellulose fiber of the eighth invention is a method for producing the sulfonated fine cellulose fiber of any one of the first to the seventh invention from pulp, the method performing a chemical treatment step of chemically treating the pulp and a fibrillation treatment step of fibrillating the pulp having been subjected to the chemical treatment step in this order, the method comprising a polymerization degree lowering treatment step of depolymerizing the pulp before the chemical treatment step.

The method for producing a sulfonated fine cellulose fiber of the ninth invention is, in the eighth invention, in the polymerization degree lowering treatment step, the pulp is brought into contact with an acid solution or an alkali solution, or is immersed in the acid solution or the alkali solution to prepare depolymerized pulp.

The method for producing a sulfonated fine cellulose fiber of the tenth invention is, in the eighth or ninth invention, in the fibrillation treatment step, the pulp having been subjected to the chemical treatment step is supplied to a fibrillation device and is fibrillated at a fibrillation pressure of 5 MPa to 60 MPa.

The method for producing a sulfonated fine cellulose fiber of the eleventh invention is, in the tenth invention, in the fibrillation treatment step, the pulp having been subjected to the chemical treatment step is repeatedly fibrillated by a fibrillation device, and the number of fibrillations is 30 passes or less.

The method for producing a sulfonated fine cellulose fiber of the twelfth invention is, in the eighth, ninth, tenth or eleventh invention, in the fibrillation treatment step, fibrillation energy of 1,000J or less is given to the pulp having been subjected to the chemical treatment step.

Advantageous Effects of Invention

Sulfonated Fine Cellulose Fiber

According to the first invention, the desired viscosity can be achieved when the fine cellulose fibers are dispersed in a dispersion liquid.

According to the second and third inventions in which the thixotropy index is a predetermined value, the degree of flexibility in handling can be improved.

According to the fourth invention in which the fine cellulose fiber is formed from a plurality of fibers with a low average polymerization degree, it is possible to more appropriately exhibit excellent viscosity. Further, since it is also possible to exhibit excellent biodegradability, the fiber can be used as a material with a low environmental burden.

According to the fifth, sixth, and seventh inventions, the transparency of a dispersion liquid of the fine cellulose fibers in a water-soluble solvent can be improved.

Method for Producing Sulfonated Fine Cellulose Fibers

According to the eighth invention including a polymerization degree lowering treatment step, the polymerization degree of the pulp fibers can be lowered before the chemical treatment. Therefore, it is possible to prepare fine cellulose fibers having a dispersion form easily changeable after the fibrillation, thereby providing sulfonated fine cellulose fibers with improved handling properties.

According to the ninth invention which enables the polymerization degree lowering treatment to be more appropriately performed, thereby easily removing colored substances during the chemical treatment, it is possible to produce highly transparent sulfonated fine cellulose fibers.

According to the tenth invention, the fibrillation treatment can be properly performed even with a low fibrillation pressure, thereby improving the operability of the fibrillation treatment step.

According to the eleventh invention, the viscosity of the sulfonated fine cellulose fibers can be controlled by adjusting the fibrillation treatment.

According to the twelfth invention, the desired sulfonated fine cellulose fibers can be obtained more appropriately by adjusting the fibrillation energy.

Brief Description of Drawings

FIG. 1 is a schematic flow diagram illustrating a method for producing sulfonated fine cellulose fibers of the present embodiment.

FIG. 2 is a diagram showing experimental results.

FIG. 3 is a diagram showing experimental results, which are images of sulfonated fine cellulose fibers with different viscosities.

FIG. 4 is a schematic explanatory view of sulfonated fine cellulose fibers of the present embodiment.

FIG. 5 is a diagram showing experimental results of a biodegradability test.

FIG. 6 is a diagram of experimental results, showing a relationship between fibrillation intensity and viscosity/TI value.

FIG. 7 is a diagram of experimental results, showing a relationship between fibrillation intensity and viscosity/TI value of a Comparative Example.

FIG. 8 is a diagram of experimental results, showing a relationship between fibrillation energy and haze value.

Description of Embodiments

Hereinbelow, embodiments of the present invention are described by referring to drawings.

The sulfonated fine cellulose fibers of the present embodiment (hereinafter simply referred to as sulfonated fine cellulose fibers) are fine cellulose fibers manufactured by fibrillating cellulose fibers, and are characterized by their capability to exhibit excellent viscosity characteristics. The sulfonated fine cellulose fiber production method of the present embodiment (hereinafter referred to as a sulfonated fine cellulose fiber production method), which is a method for producing the sulfonated fine cellulose fibers described above, is characterized by its capability to appropriately produce the sulfonated fine cellulose fibers exhibiting excellent viscosity.

The term "viscosity characteristics" used in the present specification refers to a property of exhibiting high viscosity and the desired TI value (e.g., a low TI value, a high TI value).

Hereinbelow, embodiments of the sulfonated fine cellulose fibers are described first, followed by embodiments of the sulfonated fine cellulose fiber production method.

Sulfonated Fine Cellulose Fiber

The sulfonated fine cellulose fibers are fine cellulose fibers obtained by fibrillating cellulose fibers. The sulfonated fine cellulose fiber contains several further-finer cellulose fibers (hereafter referred to as unit fibers). Specifically, the sulfonated fine cellulose fiber is a fiber formed by multiple unit fibers connected together. In the unit fibers, at least a part of hydroxyl groups (-OH groups) of the cellulose (a chain polymer formed of $\beta$ (1 → 4) glycosidically-bonded D-glucose, hereinafter may be simply referred to as a cellulose molecule) constituting the fiber is sulfonated by the sulfo group represented by the following formula (1). That is, in the sulfonated fine cellulose fiber, a part of the hydroxyl groups of the fine cellulose fiber is substituted with sulfo groups.

$$(-SO_3^-)_r \cdot Z^{r+} \qquad (1)$$

(wherein r is an independent natural number of 1 to 3; when r = 1, $Z^{r+}$ is at least one member selected from the group consisting of hydrogen ion, alkali metal cation, ammonium ion, aliphatic ammonium ion, aromatic ammonium ion and cationic polymer; when r = 2 or 3, Zr+ is at least one member selected from the group consisting of cation of an alkaline earth metal or cation of a polyvalent metal, and compounds containing two or more cationic functional groups in the molecule, such as diamine.

The sulfonated fine cellulose fiber may have other functional group(s) bonded to a part of the hydroxyl groups of the fine cellulose fiber; in particular, the sulfonated fine cellulose fiber may contain a functional group (substituent) containing sulfur, other than the sulfo group.

The following describes a case where only sulfo groups are introduced with respect to the hydroxyl groups of the cellulose fibers constituting the sulfonated fine cellulose fibers as a representative example.

Amount of Sulfo Group Introduced Into Sulfonated Fine Cellulose Fiber

The amount of sulfo groups introduced into the sulfonated fine cellulose fiber can be expressed by the amount of sulfur attributable to sulfo groups.

The amount of sulfo group to be introduced is not particularly limited. For example, the introduction amount of sulfur attributable to sulfo groups per 1g (mass) of the sulfonated fine cellulose fiber is adjusted to preferably more than 0.42 mmol/g, more preferably 0.42 mmol/g to 9.9 mmol/g, further preferably 0.5 mmol/g to 9.9 mmol/g, and further more preferably 0.6 mmol/g to 9.9 mmol/g.

Since the number of sulfur atoms in the sulfo group is 1, the ratio of the amount of sulfur introduced to the amount of sulfo group introduced is 1:1. For example, when the amount of sulfur introduced per 1g (mass) of the sulfonated fine cellulose fibers is 0.42 mmol/g, the amount of sulfo group introduced is evidently 0.42 mmol/g.

When the introduction amount of sulfur attributable to sulfo groups per 1g (mass) of the sulfonated fine cellulose fibers is 0.42 mmol/g or less, the dispersibility tends to decrease because of the strong hydrogen bond between the fibers. In contrast, when the amount of the sulfur introduced is more than 0.42 mmol/g, the dispersibility can be easily improved; further, when the amount of the sulfur introduced is 0.5 mmol/g or more, the electronic repulsion can be further increased, and thus the dispersed state can be easily and stably maintained. In other words, to homogenize the viscosity of the dispersion liquid in which the sulfonated fine cellulose fibers are dispersed at a predetermined concentration, which will be described later, the sulfur introduction amount is preferably more than 0.42 mmol/g, and more preferably 0.5 mmol/g or more. On the other hand, as the amount of the sulfur introduced becomes closer to 9.9 mmol/g, there is a concern that the crystallinity may decrease, and the costs for introducing sulfur tend to increase.

Therefore, the amount of sulfo groups to be introduced to the sulfonated fine cellulose fiber, i.e., the introduction amount of sulfur attributable to sulfo groups is adjusted to preferably more than 0.42 mmol/g and not more than 3.0 mmol/g, more preferably 0.5 mmol/g to 3.0 mmol/g, even more preferably 0.5 mmol/g to 2.0 mmol/g, further preferably 0.5 mmol/g to 1.7 mmol/g, and further more preferably 0.5 mmol/g to 1.5 mmol/g.

Also in view of the transparency of the sulfonated fine cellulose fiber, it is preferable to adjust the amount of sulfur to be introduced attributable to sulfo groups to fall within a range similar to the above range.

Method for Measuring the Amount of Sulfo Group Introduced

The amount of sulfo groups introduced into the sulfonated fine cellulose fibers can be evaluated by the introduction amount of sulfur attributable to sulfo groups or by the direct measurement of sulfo groups.

For example, the amount may be determined based on the value obtained by combusting a predetermined amount of the sulfonated fine cellulose fibers and measuring the sulfur content in the combusted material according to IEC 62321 using combustion-ion chromatography, or may be determined based on the value obtained by treating the sulfonated fine cellulose fibers with an ion exchange resin and then measuring the electrical conductivity while adding an aqueous sodium hydroxide solution dropwise thereto.

For example, the latter method for determining the amount of sulfo groups introduced is described below. More specifically, the amount may be calculated by measuring electrical conductivity, as described in the Examples below.

First, a strongly acidic ion exchange resin (Amberjet 1024 (conditioned), manufactured by Organo Corporation) at a volume ratio of 1/10 was added to a 0.2% by mass of the sulfonated fine cellulose fibers, and the mixture was subjected to a shaking treatment at least for 1 hour (treatment with an ion exchange resin). Thereafter, the mixture was poured to an about 90 to 200 μm-opening mesh, thereby separating the resin from the slurry. In the subsequent titration using an alkali, while adding 0. 5N aqueous sodium hydroxide solution to the sulfonated fine cellulose fiber-containing slurry having been through the ion exchange resin treatment, changes in electrical conductivity values were measured. With the obtained measurement data, the electrical conductivity is plotted on the vertical axis and the amount of sodium hydroxide titrated is plotted on the horizontal axis, thereby obtaining a curve and confirming the inflection point. The amount of sodium hydroxide titrated at the inflection point corresponds to the amount of the sulfo groups. By dividing the amount of sodium hydroxide at the inflection point by the amount of solids content of the sulfonated fine cellulose fibers used in the measurement, the amount of the sulfo groups, i.e., the introduction amount of sulfur attributable to the sulfo groups, can be determined.

When the sulfonated fine cellulose fibers are prepared from by fibrillating the chemically-treated sulfonated fine cellulose fibers as described later, the amount of sulfo groups introduced may be determined from the amount of sulfur introduced in the sulfonated pulp fiber before the fibrillation.

Viscosity of Sulfonated Fine Cellulose Fiber

The sulfonated fine cellulose fibers are prepared so that the appropriate viscosity can be achieved when the sulfonated fine cellulose fibers are dispersed in a dispersion liquid.

Specifically, it is sufficient that the viscosity of the dispersion liquid adjusted to have a predetermined solid concentration (e.g., 0.5% by mass) of the sulfonated fine cellulose fibers is 5,000 mPa•s or more, preferably 10,000 mPa•s or more, more preferably 15,000 mPa•s or more, and even more preferably 20,000 mPa•s or more.

Method for Measuring Viscosity

The viscosity can be measured, for example, using a Brookfield viscometer described in the Examples below.

For example, the viscosity of the sulfonated fine cellulose fibers can be measured by measuring a dispersion liquid prepared so that the solid concentration of the sulfonated fine cellulose fibers becomes 0.5% by mass using a Brookfield viscometer at a rotation speed of 6 rpm, 25°C, for 3 minutes.

The solvent used in the dispersion liquid may be a solvent soluble in water (water-soluble solvent). For example, in addition to water alone, alcohol, ketone, amine, carboxylic acid, ether, amide, or a mixture thereof may be used.

Average Fiber Width of Sulfonated Fine Cellulose Fiber

The sulfonated fine cellulose fibers are fine cellulose fibers obtained by fibrillating cellulose fibers as described above, and are very thin. Specifically, the sulfonated fine cellulose fibers are prepared to have an average fiber width of preferably 1 nm to 30 nm, more preferably 2 nm to 30 nm, when observed with a device capable of nanoscale observation, such as an electron microscope or an atomic force microscope.

When the average fiber width of the sulfonated fine cellulose fibers is more than 30 nm, the aspect ratio tends to decrease and the entanglement between fibers may be decreased, thereby decreasing the viscosity.

Therefore, the average fiber width of the sulfonated fine cellulose fibers is preferably 2 nm to 30 nm, more preferably 2 nm to 20 nm, and further preferably 2 nm to 10 nm in view of an increase in viscosity.

Further, when the average fiber width is more than 30 nm, which is close to 1/10 of the wavelength of visible light, the refraction and scattering of visible light easily occur at the interface when the fibers are combined with a matrix material. As a result, due to the scattering of visible light, the transparency (described later) tends to decrease. Therefore, in view of transparency (described later), the sulfonated fine cellulose fibers is preferably prepared so as to have the average fiber width of 20 nm or less, more preferably 10 nm or less. In particular, when the sulfonated fine cellulose fibers are prepared to have the average fiber width of 10 nm or less, scattering of visible light can be reduced; therefore, sulfonated fine cellulose fibers having high transparency can be obtained.

Method for Measuring Average Fiber Width

The average fiber width of the sulfonated fine cellulose fiber can be measured using a known technique.

The average fiber width can be measured, for example, using a scanning probe microscope described in the Examples below. Specifically, the sulfonated fine cellulose fibers are dispersed in a solvent such as pure water to adjust a mixed solution having a predetermined mass%. The mixed solution is spin-coated on a silica substrate coated with PEI (polyethyleneimine), and the sulfonated fine cellulose fibers on the silica substrate are observed. As an observation method, for example, a scanning probe microscope (for example, SPM-9700 manufactured by Shimadzu Corporation) can be used. The average fiber width of the sulfonated fine cellulose fibers can be determined by randomly selecting 20 sulfonated fine cellulose fibers in the obtained observation image, measuring the individual fiber widths, and averaging the measured widths.

As described above, the above structure allows the sulfonated fine cellulose fibers to exhibit excellent viscosity when dispersed in a dispersion liquid.

Fiber Length of Sulfonated Fine Cellulose Fibers and Average Polymerization Degree of Unit Fiber

As described above, the sulfonated fine cellulose fiber is a fiber formed by a plurality of unit fibers connected together. The number of connected unit fibers is not particularly limited, insofar as they are connected to have a length to exhibit the viscosity described above.

On the other hand, the average fiber length of the unit fibers constituting the sulfonated fine cellulose fiber, i.e., the average polymerization degree of the unit fibers, is adjusted to be around the levelling-off degree of polymerization of cellulose, which is described later. More specifically, the unit fibers are prepared so as to have an average polymerization degree of preferably 100 to 350, more preferably 200 to 350, further preferably 250 to 350.

The average fiber length of the unit fibers can be calculated from the average polymerization degree. When the length of a single glucose molecule in the axis-wise direction is about 5A (about 0.5 nm) and when the polymerization degree of the sulfonated fine cellulose fibers is 350, it is theoretically possible to prepare sulfonated fine cellulose fibers having a fiber length of about 175 nm (about 0.175 $\mu$m). In other words, when the average polymerization degree of the unit fibers is 350, the sulfonated fine cellulose fibers automatically have an average fiber length of 175 nm or more, which is the average fiber length of the unit fibers.

When the polymerization degree of the unit fibers is within the above-specified range, the fibers can be easily decomposed by microorganisms or the like, thus improving biodegradability. Specifically, by bringing microorganisms or the like into contact with sulfonated fine cellulose fibers, it is possible to improve the degradation efficiency of cellulolytic enzymes (cellulases) having endoglucanase or exoglucanase activities that cleave the cellulose molecular chains of the unit fibers constituting the sulfonated fine cellulose fibers. In particular, it is possible to increase the attacking points of the cellulolytic enzymes with exoglucanase activity that cleave the cellulose molecular chains from the terminal side of the cellulose molecular chain. This allows enzymatic degradation to proceed more efficiently by microorganisms or the like, thereby improving the biodegradability of sulfonated fine cellulose fibers.

For example, the cellulolytic enzymes (cellulases) described above can be used as an indicator of biodegradability. This evaluation method includes, for example, the degradation measurement method by an enzymatic treatment described in the Examples below. The method is not limited to the method described above and, for example, the method of measuring the biochemical oxygen demand (BOD) and the like may be used.

The evaluation method using a cellulolytic enzyme (cellulase) is performed, for example, by suspending sulfonated fine cellulose fibers in an acetate buffer solution (pH 5.0), and then adding a cellulase obtained from a commercial supplier (e.g., cellulase from Nacalai Tesque, Inc.), followed by degradation for a certain period of time. Thereafter, the degraded matter is filtered, and the weight reduction rate of the recovered fibers is measured for evaluation. The evaluation may also be performed by quantifying the degradation products such as glucose contained in the filtrate resulting from the filtration by way of liquid chromatography such as high-performance liquid chromatography (HPLC).

Therefore, by setting the polymerization degree of the unit fibers within the above predetermined range, it is possible to make the fine cellulose fibers function as a thickener while appropriately exhibiting biodegradability, thereby further improving the degree of flexibility in handling the fine cellulose fibers.

For example, by incorporating the fibers as a thickener in cosmetics, paint products, food products and the like as a thickener, it is possible to improve the thickening properties of the products while improving the biodegradability after use, thus reducing the environmental burden.

The "biodegradability" in the present specification means detection of soluble monosaccharides by enzymatic degradation.

Generally, if an increase in viscosity of fibers is desired, the fibers are prepared to have a long fiber length. In other words, the conventional techniques are aimed at increasing the polymerization degree of a single fiber as much as possible so as to improve viscosity. On the other hand, the inventors of the present invention found, for the first time, that the viscosity of sulfonated fine cellulose fibers can be improved by connecting multiple fibers, even when each fiber (unit fiber) has a short length. In other words, the inventors accomplished the present invention based on an idea that is completely opposite from the general idea of increasing the length of each fiber to improve the viscosity of the fibers. In other words, the inventors of the present invention found for the first time that the viscosity of the dispersion liquid in which the sulfonated fine cellulose fibers are dispersed does not depend on the polymerization degree, but on the fiber length resulting from connecting many unit fibers. Based on this finding, the inventors completed the present invention.

Moreover, the inventors of the present invention found for the first time that the viscosity of sulfonated fine cellulose fibers can be controlled by adjusting the amount of connection of fine fibers (unit fibers) constituting the sulfonated fine cellulose fibers. Based on this finding, the inventors completed the present invention.

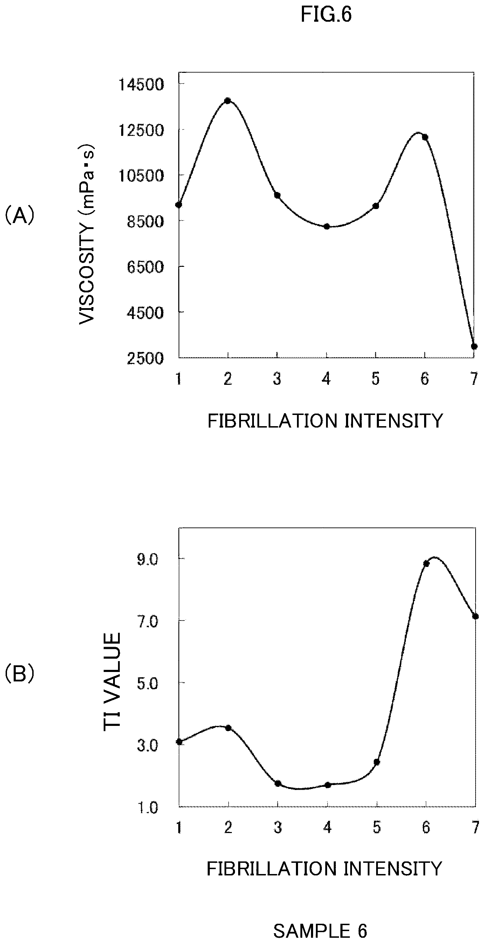

Furthermore, the inventors of the present invention found for the first time that, by preparing the unit fibers of sulfonated fine cellulose fibers to have a polymerization degree near the levelling-off degree of polymerization, it is possible to vary the thixotropy even when the viscosity of the dispersion liquid is nearly the same. Based on this finding, the inventors completed the present invention. More specifically, the inventors of the present invention found for the first time that, by preparing the unit fibers of sulfonated fine cellulose fibers with a polymerization degree near the levelling-off degree of polymerization, it is possible to exhibit predetermined viscosity characteristics (high viscosity, predetermined thixotropy). Based on this finding, the inventors completed the present invention (see FIGS. 6(A) and 6(B)).

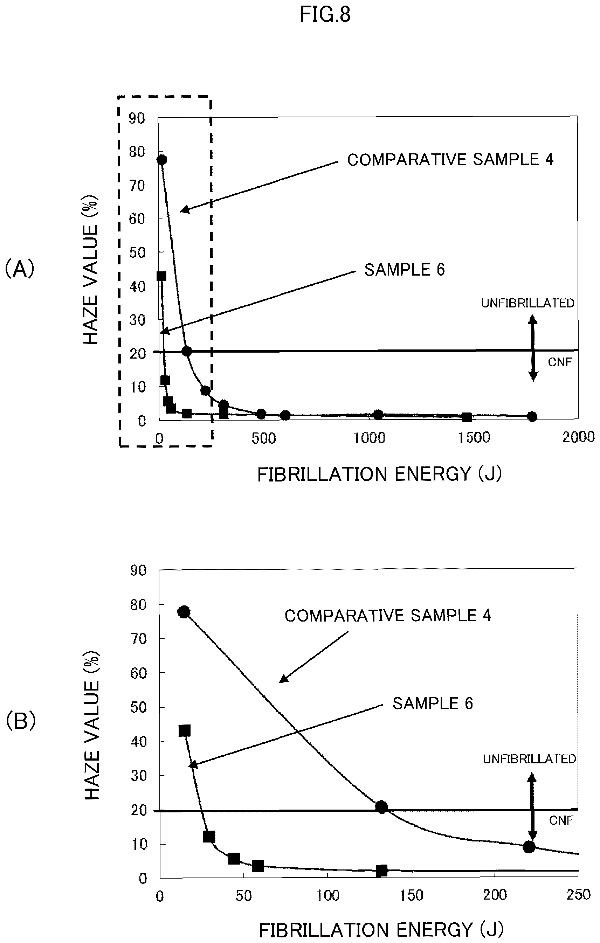

Further, the inventors of the present invention found for the first time that, by preparing the unit fibers of sulfonated fine cellulose fibers with a polymerization degree near the levelling-off degree of polymerization, it is possible to maintain the desired productivity of the sulfonated fine cellulose fibers having predetermined viscosity characteristics and high transparency, even when the energy required for the fibrillation treatment of the sulfonated pulp fibers is low. Based on this finding, the inventors completed the production method of the present invention (see FIG. 8).

Method for Measuring Average Polymerization Degree of Unit Fibers

The average polymerization degree of the unit fibers constituting the sulfonated fine cellulose fibers can be measured by a known measurement method (e.g., the method in cupri-ethylene -diamine solution), such as the method described below. The average polymerization degree can be measured, for example, using the method according to JIS P 8215 described later, or the method based on JIS P 8215 used in the Examples below.

Further, for example, when the method in cupri-ethylene -diamine solution is performed, the sulfonated fine cellulose fibers are dissolved in 0.5 M cupri-ethylene -diamine solution. Then, the viscosity of the solution in which the sulfonated fine cellulose fibers are dissolved is measured by the viscosity method, thereby determining the average polymerization degree of the unit fibers in the state where the unit fibers constituting the sulfonated fine cellulose fibers are unconnected.

The levelling-off degree of polymerization mentioned above means as follows. When cellulose is hydrolyzed, the polymerization degree decreases by selective depolymerization of so-called an amorphous region, which is a non-crystalline region other than the region where acids can permeate. It is known that the polymerization degree decreases rapidly in the initial stage of decomposition, and then becomes slower once a certain degree of polymerization is achieved (INDUSTRIAL AND ENGINEERING CHEMISTRY, Vol. 42, No. 3, p. 502-507 (1950)). Such a constant average polymerization degree is called a "levelling-off degree of polymerization", and it is believed that once the polymerization degree comes to the levelling-off degree of polymerization, it will not fall below the levelling-off degree of polymerization even if the hydrolysis time is extended.

For example, by hydrolyzing NBKP (Needle Bleached Kraft Pulp) using 2.5 N hydrochloric acid at the boiling temperature for 15 minutes, followed by measurement according to the viscosity method (method in cupri-ethylene -diamine solution), it is possible to determine the levelling-off degree of polymerization of woody cellulose fibers. The levelling-off degree of polymerization is determined when the polymerization degree comes to about 200 to 300 under the above conditions.

Thixotropy Index (TI Value) of Sulfonated Fine Cellulose Fibers

The sulfonated fine cellulose fibers are preferably prepared so that the viscosity described above can be exhibited when dispersed in a dispersion liquid and that an appropriate thixotropy index (TI value) can be exhibited. In other words, the sulfonated fine cellulose fibers are preferably prepared to exhibit predetermined viscosity characteristics (high viscosity, predetermined TI value) when dispersed in a dispersion liquid.

The TI value is an evaluation based on the viscosity that occurs when the external stress is applied to the above dispersion. A high TI value means that the dispersion liquid has a property of easily exhibiting fluidity when a predetermined force (energy) is applied. A low TI value means that the dispersion liquid is unlikely to exhibit fluidity when a predetermined force (energy) is applied.

Based on this property, for sulfonated fine cellulose fibers with the same viscosity, i.e., a similar fiber length (in other words, sulfonated fine cellulose fibers in which unit fibers having a similar polymerization degree are connected (e.g., linearly connected)), the following can be inferred from the viewpoint of fiber state.

In the static state of the dispersion liquid, the sulfonated fine cellulose fibers are entangled with each other. The entanglement develops a high viscosity.

Then a predetermined force is applied to the dispersion liquid. In this case, it is inferred that sulfonated fine cellulose fibers having a property with a low TI value have a structure unlikely to cause a decrease in viscosity even when predetermined energy is applied. In other words, the sulfonated fine cellulose fibers exhibiting high viscosity and a low TI value have a structure in which the entanglement of fibers does not easily collapse (i.e., the entanglement of fibers does not easily come apart). On the other hand, it is inferred that sulfonated fine cellulose fibers having a property with a high TI value have a structure more likely to cause a decrease in viscosity when predetermined energy is applied.

In other words, the sulfonated fine cellulose fibers having high viscosity and a high TI value have a structure in which the entanglement of fibers easily collapses (i.e., the entanglement of fibers easily comes apart).

More specifically, the TI value can presumably be used as one of the structural indicators for sulfonated fine cellulose fibers exhibiting similar viscosities.

For example, for sulfonated fine cellulose fibers with an entanglement that does not easily collapse even when a predetermined force is applied to the dispersion liquid thereof, that is, the dispersion liquid has small flowability even when a predetermined force is applied (sulfonated fine cellulose fibers with a low TI value), a dispersion liquid adjusted such that the solid concentration of the sulfonated fine cellulose fibers to be a predetermined value (e.g., 0.5 % by mass) has a TI value of 1.0 to 4.0 (not including 4.0), preferably 1.0 to 3.5 or less, and more preferably 1.0 to 3.0 or less. The fibers with a TI value in this range can be regarded as sulfonated fine cellulose fibers with a structure in which the entangled state is strongly maintained.

For example, for sulfonated fine cellulose fibers with an entanglement that easily collapses when a predetermined force is applied to the dispersion liquid thereof, that is, the dispersion liquid has large flowability as compared with the above sulfonated fine cellulose fibers exhibiting a low TI value (sulfonated fine cellulose fibers with a high TI value), a dispersion liquid adjusted such that the solid concentration of the sulfonated fine cellulose fibers to be a predetermined value (e.g., 0.5 % by mass) has a TI value of 4.0 or more, preferably 5.0 or more, and more preferably 7.0 or more. The fibers with a TI value in this range can be regarded as sulfonated fine cellulose fibers with a structure in which the entangled fibers more easily come apart.

By preparing the sulfonated fine cellulose fibers with a TI value higher than the above value, the flowability of the dispersion liquid can be improved when the external stress is applied. As a result, it is possible to easily ensure the dispersibility of the sulfonated fine cellulose fibers in the dispersion liquid or allow easy dilution of the dispersion liquid, thus improving the handling property.

Method for Measuring Thixotropy Index (TI Value)

The thixotropy index (TI value) can be measured, for example, using a Brookfield viscometer described in the Examples below. In summary, the thixotropy index can be calculated by measuring a dispersion liquid prepared so that the solid concentration of the sulfonated fine cellulose fibers becomes 0.5% by mass using a Brookfield viscometer at rotation speeds of 6 rpm and 60 rpm and at 25°C, calculating each viscosity, and determining each viscosity ratio (viscosity at a rotation speed of 6 rpm/viscosity at a rotation speed of 60 rpm).

Transparency of Sulfonated Fine Cellulose Fibers

By preparing sulfonated fine cellulose fibers so that the average fiber width of the sulfonated fine cellulose fibers falls within the above range, it is possible to obtain sulfonated fine cellulose fibers having excellent transparency. The transparency described above when the sulfonated fine cellulose fibers are dispersed in a dispersion liquid can be evaluated as follows.

In the present specification, "transparency" includes both or either of transparency and turbidity of a liquid. Specifically, in the evaluation of transparency, the turbidity of the liquid can be more appropriately evaluated by the haze value, and the transparency of the liquid can be more appropriately evaluated by the total light transmittance. The respective evaluation methods are specifically described below.

Haze Value

The haze value of a dispersion liquid in which sulfonated fine cellulose fibers are dispersed at a predetermined concentration can be measured as follows.

First, a dispersion liquid in which sulfonated fine cellulose fibers are dispersed in a water-soluble solvent at a solid concentration of 0.1% by mass to 20% by mass is prepared. If the haze value of the dispersion liquid is 20% or less, it can be regarded that the sulfonated fine cellulose fibers can exhibit transparency with little turbidity. On the contrary, if the haze value of the dispersion liquid prepared at a solid concentration within the above range is more than 20%, the sulfonated fine cellulose fibers can be regarded as more difficult to appropriately exhibit transparency.

For example, when preparing the dispersion liquid in which the sulfonated fine cellulose fibers are dispersed so that the solid concentration becomes 0.2% by mass to 0.5% by mass, if the haze value of the dispersion liquid is 20% or less, the sulfonated fine cellulose fibers have little turbidity and can appropriately exhibit transparency. In particular, to properly suppress the turbidity of the dispersion liquid in which the sulfonated fine cellulose fibers are dispersed at a predetermined concentration, the dispersion liquid is preferably prepared so that the haze value is 15% or less, more preferably 10% or less.

Therefore, in view of exhibiting less turbidity and transparency, the sulfonated fine cellulose fibers are preferably prepared such that the haze value of a dispersion liquid prepared to have a solid concentration of 0.1% by mass to 20% by mass is 20% or less; more preferably, the sulfonated fine cellulose fibers are prepared such that the haze value of the dispersion liquid prepared to have a solid concentration of 0.2% by mass to 0.5% by mass is 15% or less, more preferably 10% or less.

Method for Measuring Haze Value

The haze value can be measured, for example, using a spectral haze meter or a spectrophotometer according to JIS K 7105 described in the Examples below. In summary, the sulfonated fine cellulose fibers are dispersed in the dispersion liquid described above at a predetermined solid concentration. Then, by measuring the dispersion liquid using a spectral haze meter or a spectrophotometer according to JIS K 7105, the haze value of the sulfonated fine cellulose fibers can be determined.

Total Light Transmittance

The total light transmittance of a dispersion liquid in which sulfonated fine cellulose fibers are dispersed at a predetermined solid concentration can be measured as follows.

First, a dispersion liquid in which sulfonated fine cellulose fibers are dispersed in a water-soluble solvent at a solid concentration of 0.1% by mass to 20% by mass is prepared. If the total light transmittance of the dispersion liquid is 90% or more, it can be regarded that the sulfonated fine cellulose fibers can exhibit high transparency. On the contrary, if the total light transmittance of the dispersion liquid prepared at a solid concentration within the above range is less than 90%, the sulfonated fine cellulose fibers can be regarded as more difficult to exhibit appropriate transparency.

For example, when preparing the dispersion liquid in which the sulfonated fine cellulose fibers are dispersed such that the solid concentration becomes 0.2% by mass to 0.5% by mass, if the total light transmittance of the dispersion liquid is 90% or more, the sulfonated fine cellulose fibers can appropriately exhibit high transparency. In particular, to properly exhibit the transparency of the dispersion liquid in which the sulfonated fine cellulose fibers are dispersed at a predetermined concentration, the dispersion liquid is preferably prepared so that the total light transmittance is 95% or more.

Therefore, in view of exhibiting high transparency, the sulfonated fine cellulose fibers are preferably prepared such that the total light transmittance of the dispersion liquid prepared to have a solid concentration of 0.1% by mass to 20% by mass is 90% or more; more preferably, the sulfonated fine cellulose fibers are preferably prepared such that the total light transmittance of the dispersion liquid prepared to have a solid concentration of 0.2% by mass to 0.5% by mass is 90% or more, more preferably 95% or more.

Method for Measuring Total Light Transmittance

The total light transmittance can be measured, for example, using a spectral haze meter or a spectrophotometer according to JIS K 7105 described in the Examples below. In summary, first, the sulfonated fine cellulose fibers are dispersed in the dispersion liquid described above at a predetermined solid concentration. Then, the total light transmittance of the sulfonated fine cellulose fibers can be determined by measuring the dispersion liquid using a spectral haze meter or a spectrophotometer according to JIS K 7105.

The solvent of the dispersion liquid to be used for the measurements of the haze value or the total light transmittance is not particularly limited insofar as it is a solvent soluble in water (water-soluble solvent) similarly to the above-described aqueous solvent to be used for the viscosity measurement. For example, the water-soluble solvent may be water alone, alcohol, ketone, amine, carboxylic acid, ether, amide, and the like, which may be used solely or in a combination of two or more.

Color of Sulfonated Fine Cellulose Fibers

The color of the sulfonated fine cellulose fibers can be evaluated, for example, by measuring L*, a*, and b* according to JIS Z 8781-4 of the dispersion liquid in which the sulfonated fine cellulose fibers are dispersed at a predetermined concentration, as described in the examples below.

Specifically, first, a dispersion liquid is prepared by dispersing sulfonated fine cellulose fibers in a water-soluble solvent so that the solid concentration is 0.1% by mass to 20% by mass. Then, the L*a*b* color space of the prepared dispersion liquid is measured.

If the measured L*a*b* color space satisfies at least one of the following numerical ranges, the sulfonated fine cellulose fibers can be regarded as having no color and capable of exhibiting high transparency.

L* is 95% or more.

a* is not less than -5 and not more than +5.

b* is not less than -5 and not more than +5.

In particular, by preparing the sulfonated fine cellulose fibers so that a* is not less than -2 and not more than +2 and b* is not less than -2 and not more than +2, the sulfonated fine cellulose fibers can be regarded as capable of exhibiting higher transparency.

In the present specification, "transparency" includes both or either of transparency and turbidity of a liquid. Regarding the transparency evaluation, the turbidity of the liquid can be more appropriately evaluated by the haze value, and the transparency can be more appropriately evaluated by the total light transmittance. The clarity, i.e., the color in terms of transparency, can be appropriately evaluated by L*a*b* color space.

Method for Producing Sulfonated Fine Cellulose Fibers

The method for producing sulfonated fine cellulose fibers is characterized by the depolymerization of pulp as described above.

Since cellulose fibers constituted of pulp are tightly bonded to each other by hydrogen bonds, producing fine cellulose fibers generally requires a large number of fibrillation cycles with a high fibrillation pressure during the fibrillation step, thereby requiring great energy and labor.

On the other hand, the fiber length of the obtained fine cellulose fibers is generally decreased by a large number of fibrillation cycles at a high fibrillation pressure. It is assumed that, the shorter the fiber length, i.e., the lower the polymerization degree of the fine cellulose fibers, the fibers are more severely damaged, thereby decreasing the viscosity of the resulting fine cellulose fibers.

Therefore, as a conventional technology for improving the viscosity of the fine cellulose fibers, a production method of performing a fibrillation step with a less processing volume, a lower fibrillation pressure, and more frequent fibrillation cycles has previously been proposed.

However, this previously-known production method cannot be regarded as practical because the amount of fine cellulose fibers obtained is extremely small and the processing efficiency in the fibrillation treatment step is extremely low.

That is, in previously-known technologies, there is a trade-off relationship between preparing fibers having high viscosity and improving productivity, and no technology to efficiently and appropriately produce fibers with high viscosity has not been developed.

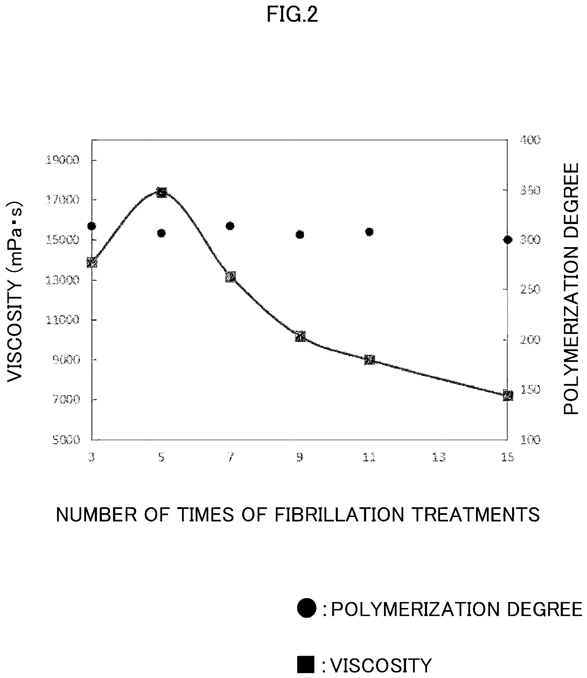

On the other hand, as mentioned above, the inventors of the present invention discovered, for the first time, a method based on an idea that is completely opposite to the previously-known idea, i.e., a method in which the pulp is depolymerized before being subjected to the fibrillation treatment step to decrease the polymerization degree of fibers on purpose, and the resulting pulp having a low polymerization degree is then subjected to the fibrillation treatment step, thereby easily and efficiently and with less energy producing sulfonated fine cellulose fibers that exhibit excellent viscosity to the extent that cannot be expected from the previously-known technology (see Table 2, FIG. 2). Moreover, the inventors also discovered for the first time that the sulfonated fine cellulose fibers thus prepared are less damaged. In other words, the inventors achieved the method for producing the sulfonated fine cellulose fibers of the present invention based on findings different from the conventional idea that the fiber length of fine cellulose fiber depends on the polymerization degree.

Further, as a solution to the problems described above, the inventors discovered for the first time a method of treating the pulp so that the polymerization degree is reduced, thereby preparing CNF (sulfonated fine cellulose fiber) that exhibits high viscosity at low energy that cannot be assumed in the conventional technologies without decreasing productivity. The step of treating the pulp so that the polymerization degree is reduced corresponds to a polymerization degree lowering treatment described later.

Moreover, the inventors discovered for the first time that sulfonated fine cellulose fibers with different structures can be prepared by adjusting the conditions of the fibrillation treatment by subjecting the pulp to the polymerization degree lowering treatment described later.

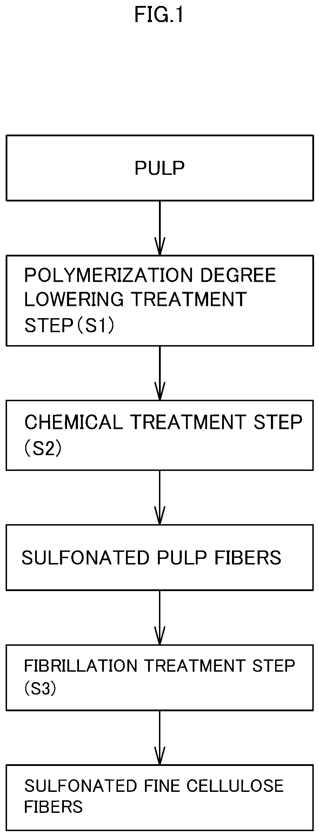

FIG. 1 shows the outline of the method for producing sulfonated fine cellulose fibers. First, a polymerization degree lowering treatment step S1 in which a fiber raw material (e.g., pulp) containing cellulose is subjected to depolymerization is performed. Then, the pulp is subjected to a chemical treatment step S2, followed by a fibrillation treatment step S3 to fibrillate the resulting pulp containing chemically-treated sulfonated pulp fibers, thereby producing sulfonated fine cellulose fibers. These steps are specifically described below.

In this specification, a "fiber raw material" refers to a fibrous raw material such as pulp containing cellulose molecules, and "pulp" refers to an aggregate of multiple pulp fibers. The "pulp fiber" refers to an aggregate of a plurality of cellulose fibers. The cellulose fiber refers to an aggregate of a plurality of fine fibers (e.g., microfibrils, or the like). The "fine fiber" refers to an aggregate of cellulose molecules (hereinafter may simply be referred to as cellulose), which is a chain polymer formed of $\beta$ (1→4) glycosidically-bonded D-glucose. The details of the fiber raw material are described later.

Polymerization Degree Lowering Treatment Step S1

The polymerization degree lowering treatment step S1 is a process of depolymerizing a fiber raw material containing cellulose. Specifically, the polymerization degree lowering treatment step S1 includes a contact step in which a polymerization degree lowering agent is brought into contact with a fiber raw material such as pulp, and a reaction step of lowering the polymerization degree of the fiber raw material resulting from the contact step.

"Depolymerization" refers to the phenomenon of decreasing the polymerization degree of pulp or decreasing the molecular weight of the pulp.

Contact Step in Polymerization Degree Lowering Treatment Step S1

The contact step in the polymerization degree lowering treatment step S1 is a step of bringing a polymerization degree lowering agent into contact with a fiber raw material. The contact step is not particularly limited insofar as it is capable of bringing a polymerization degree lowering agent into contact with a fiber raw material. For example, it is possible to immerse a fiber raw material (wood pulp, or the like) in a treatment solution containing a polymerization degree lowering agent that lowers the polymerization degree so as to impregnate the fiber raw material with the polymerization degree lowering agent, or applying the treatment solution to the fiber raw material.

Among these methods, the method of impregnating the fiber raw material with the treatment solution containing the polymerization degree lowering agent by immersing the fiber raw material in the treatment solution is preferable because the method is capable of uniformly bringing the polymerization degree lowering agent into contact with the fiber raw material.

Treatment Solution Used in Polymerization Degree Lowering Treatment Step S 1

The treatment solution used in the polymerization degree lowering treatment S1 is not particularly limited, insofar as it contains a polymerization degree lowering agent capable of decreasing the polymerization degree.

Examples of polymerization degree lowering agents include alkaline agents such as sodium hydroxide, potassium hydroxide, sodium hydrogencarbonate, and the like, which are alkaline when made into an aqueous solution, and acid agents such as hydrochloric acid, nitric acid, and the like, which are acidic when made into an aqueous solution. A suitable agent may be used depending on the application of the sulfonated fine cellulose fibers to be prepared.

In particular, by using an alkaline agent as a polymerization degree lowering agent, it is possible to more easily remove hemicellulose components from the fiber raw material when NBKP or LBKP described later are used as a fiber raw material. This makes it easier to prepare sulfonated fine cellulose fibers with less coloring, less colored to be yellowish or the like, and therefore are more transparent.

Amount of Contact with Treatment Solution

The amount of contact with the treatment solution is not particularly limited, insofar as the polymerization degree is decreased.

For example, when a treatment solution prepared by dissolving a polymerization degree lowering agent in water at a concentration of 1 M is used, 100 parts by mass of the treatment solution is brought into contact with the pulp of 1 part by mass to 500 parts by mass in terms of dry weight.

Reaction Step in Polymerization Degree Lowering Treatment Step S1

The reaction step in the polymerization degree lowering treatment step S1 is a step of depolymerizing the fiber raw material having been in contact with the treatment solution.

The reaction step is not particularly limited insofar as it is capable of depolymerization of a fiber raw material using a treatment solution. For example, the depolymerization may be performed by allowing the fiber raw material having been in contact with the treatment solution to stand as it is under a normal temperature and a normal pressure or by heating the fiber raw material at a predetermined temperature. The former method is desirable in view of preventing excessive depolymerization, while the latter method is desirable in view of increasing the efficiency in the polymerization degree lowering treatment.

Washing Step after Polymerization Degree Lowering Treatment Step S1

A washing step may be performed to wash the fiber raw material after the polymerization degree lowering treatment step S1.

When alkaline agents are used as the polymerization degree lowering agent to be incorporated in the treatment solution used in the polymerization degree lowering treatment step S1, if excessive alkaline agents remain, the sulfonating agent used in the chemical treatment step S2 described later is consumed to neutralize the alkaline agent, and the chemical treatment step S2 may not be properly performed. For this reason, when an alkaline agent is used as a polymerization degree lowering agent, the fiber raw material is preferably washed before being subjected to the chemical treatment step S2. As a result, it is possible to improve the efficiency in the process of bringing the fiber raw material into contact with the sulfonating agent in the chemical treatment step S2, thereby properly performing the treatment.

The washing step is not particularly limited insofar as it is capable of washing the fiber raw material having been subjected to the polymerization degree lowering treatment step S1 to be substantially neutral. For example, a method of washing the fiber raw material having been subjected to the polymerization degree lowering treatment step S 1 with pure water until the fiber raw material becomes substantially neutral can be employed.

Chemical Treatment Step S2

The chemical treatment step S2 performs a contact step of bringing cellulose fibers of a fiber raw material containing cellulose such as depolymerized pulp into contact with a sulfonating agent having sulfo groups and urea, and a reaction step of introducing sulfo groups into at least a part of the hydroxyl groups of the cellulose fibers of the pulp resulting from the contact step.

Contact Step of Chemical Treatment Step S2

The contact step in the chemical treatment step S2 is a step of bringing a sulfonating agent and urea into contact with a fiber raw material containing cellulose. The contact step is not particularly limited insofar as the method is capable of causing the above contact.

For example, a method of, for example, impregnating the fiber raw material (depolymerized pulp, the same hereinafter) with a reaction solution in which a sulfonating agent and urea coexist by immersing the fiber raw material in the reaction solution, a method of applying the reaction solution onto the fiber raw material, or a method of applying a sulfonating agent and urea separately to the fiber raw material or impregnating the fiber raw material separately with a sulfonating agent and urea may be used.

Among these methods, the method of impregnating the fiber raw material with the reaction solution by immersing the fiber raw material in the reaction solution is preferable because the method enables the sulfonating agent and urea to be uniformly brought into contact with the fiber raw material.

(Mixing Ratio of Reaction Solution)

When the method of impregnating the fiber raw material with the reaction solution by immersing the fiber raw material in the reaction solution is employed, the mixing ratio of the sulfonating agent and urea contained in the reaction solution is not particularly limited.

For example, it is possible to adjust the amounts of the sulfonating agent and urea to have a concentration ratio (g/L) of 4:1 (1:0.25), 2:1 (1:0.5), 1:1, or 1:2.5.

By dissolving the sulfonating agent and urea in water so that their concentrations are adjusted to 200g/L and 100g/L, respectively, a reaction solution containing a sulfonating agent and urea at a concentration ratio (g/L) of 2:1 can be prepared. In other words, by adjusting the amount of urea to 50 parts by weight with respect to 100 parts by weight of sulfamic acid, a reaction solution containing a sulfonating agent and urea at a proportion of 2:1 can be prepared.

Further, by dissolving the sulfonating agent and urea in water so that their concentrations are adjusted to 200g/L and 500g/L, respectively, a reaction solution containing a sulfonating agent and urea at a concentration ratio (g/L) of 1:2.5 can be prepared.

Amount of Contact with Reaction Solution

The amount of the reaction solution to be brought into contact with the fiber raw material is also not particularly limited.

For example, the amount may be adjusted so that the amount of the sulfonating agent contained in the reaction solution is 1 part by weight to 20,000 parts by weight with respect to 100 parts by dry weight of the fiber raw material, and the amount of the urea and/or the derivative thereof contained in the reaction solution is 1 part by weight to 100,000 parts by weight with respect to 100 parts by dry weight of the fiber raw material, in a state in which the reaction solution and the fiber raw material are in contact with each other.

Reaction Step in Chemical Treatment Step S2

The reaction step in the chemical treatment step S2 is a step of substituting the hydroxyl groups of the cellulose fibers contained in the fiber raw material with the sulfo groups of the contacted sulfonating agent as described above so as to introduce the sulfo groups into the cellulose fibers contained in the fiber raw material.

This reaction step is not particularly limited insofar as the method can cause a sulfonation reaction that substitutes hydroxyl groups of the cellulose fibers with sulfo groups.

For example, by subjecting the fiber raw material in contact with (e.g., impregnated with) the reaction solution to a reaction step as it is and heating the fiber raw material at a predetermined temperature, sulfo groups can be appropriately introduced into the cellulose fibers contained in the fiber raw material.

Specifically, in the reaction step, the sulfonation reaction can proceed while supplying heat to the supplied fiber raw material. More specifically, by performing heating in the reaction step, the sulfonation reaction for introducing sulfo groups with respect to the hydroxyl groups of the cellulose fibers contained in the fiber raw material can be performed while drying the fiber raw material in which the reaction solution is impregnated and/or adhered. In this case, since the amount of water in the sulfonation reaction (the amount of water in the reaction solution adhered to the fiber raw material or the like) can be reduced, the sulfonation reaction can be performed with less influence of the moisture (for example, reaction failure, hydrolysis, or the like).

Therefore, by performing the reaction of the reaction step while performing heating at a predetermined temperature, it is possible to improve the efficiency of the introduction of sulfo groups with respect to the hydroxyl groups of the cellulose fibers while suppressing damage to the cellulose fibers contained in the supplied fiber raw material. In addition, as described later, sulfo groups can be appropriately introduced with respect to predetermined hydroxyl groups of the cellulose fibers in a short time.

The fiber raw material to be supplied in the reaction step is not particularly limited insofar as it contains moisture as described above. For example, the fiber raw material to be supplied to the reaction step may be a fiber raw material having a high moisture content resulting from the contact step; the fiber raw material may be subjected to a dehydration treatment or the like to lower the moisture content before being supplied to the reaction step; or the fiber raw material may be subjected to a drying treatment or the like to further lower the moisture content. In other words, the fiber raw material to be supplied to the reaction step is not particularly limited insofar as the fiber raw material is in a state other than a non-moisture state, that is, insofar as the fiber raw material is in a non-absolute dry state having a moisture content of 1% or more.

In the present specification, such a fiber raw material in a non-absolute dry state having a moisture content of 1% or more is referred to as a moisture-containing state (i.e., a wet state), and a fiber raw material in a state of being impregnated with a reaction solution, a fiber raw material in a state of being dehydrated to some extent, and a fiber raw material in a state of being dried to some extent are also referred to as a fiber raw material in a wet state.

In the present specification, the term "absolute dry" refers to a state in which the moisture content is lower than 1%. The drying treatment step to ensure an absolute dry state refers to a step of performing a drying treatment by reducing the pressure using a desiccant or the like containing a drying agent such as calcium chloride or diphosphorus pentoxide. In other words, the step of drying the fiber raw material before having been supplied to the reaction step in the specification of the present application is not a drying treatment step to ensure an absolute dry state.

The state in which the fiber raw material is being impregnated with the reaction solution when it is supplied to the reaction step may refer to, for example, a state where the fiber raw material is in contact with the reaction solution with the moisture dripping, i.e., the fiber raw material has a moisture content of nearly 100%.

The state in which the moisture is removed to some extent from the fiber raw material resulting from the contact step before being supplied to the reaction step refers to a state in which the fiber raw material is not in an absolute dry state (which is a state in which the moisture content of the fiber raw material to be supplied to the reaction step is lower than 1%) when the fiber raw material is supplied to the reaction step, and is not particularly limited insofar as the moisture content is 1% or more. This method of removing moisture from the fiber raw material to some extent after the contact step can be performed by, for example, dehydrating the fiber raw material resulting from the contact step as described above, by drying the fiber raw material after dehydration or the like, or by directly drying the fiber raw material resulting from the contact step.

Since the moisture is removed to some extent, this case has the advantage of improved handling property and operability. The dehydration treatment may be performed by, for example, a usual treatment method, such as suction dehydration or centrifugal dehydration, and the moisture content of the fiber raw material to be supplied to the reaction step may be adjusted to about 20% to 80%. For example, a drying treatment may be performed using a dryer or other general devices, and the moisture content of the fiber raw material after the drying treatment may be adjusted to not less than 1% and less than 20%.

The moisture content in this specification can be calculated by the following formula.

$$\text{Moisture content (\%)} = 100 \cdot (\text{weight (g) of solids content of the fiber raw material/weight (g) of the fiber raw material at the time of moisture content measurement after the contact step}) \times 100$$

The weight (g) of solids content of the fiber raw material is the dry weight of the fiber raw material to be measured. For example, the weight of solids content may be a value obtained by drying 2 g (dry weight) of a fiber raw material, which is to be supplied to the reaction step, having a moisture content of about 50%, i.e., containing substantially the same amount of moisture, in advance at 105°C for 2 hours using a dryer, and measuring the dried fiber raw material.

The weight (g) of the fiber raw material at the time of moisture content measurement after the contact step is a weight of the fiber raw material at the time of measurement after the contact step, and includes, for example, the weight of the fiber raw material when supplied to the reaction step described above, the weight of the fiber raw material after the dehydration treatment after the contact step, the weight of the fiber raw material after the drying treatment, or the like.

Reaction Temperature in Reaction Step

The reaction temperature in the reaction step is not particularly limited insofar as it enables sulfo groups to be introduced into the cellulose fibers constituting the fiber raw material while suppressing thermal decomposition or hydrolysis reaction of the fibers.

Specifically, any temperature to directly or indirectly heat the fiber raw material after the contact step while satisfying the above requirements can be used. Examples of the methods for such heating include, for example, those using a known dryer, a vacuum-dryer, a microwave heating apparatus, an autoclave, an infrared heating apparatus, a hot press method using a hot press machine (for example, AH-2003C manufactured by AS ONE Corporation), and the like. In particular, since a gas may be generated in the reaction step, it is preferable to use a circulation air dryer.

The shape of the fiber raw material after the contact step is not particularly limited. However, the reaction can be easily uniformly advanced by heating the fiber raw material, for example, in a sheet shape or in a relatively disaggregated state using the above-mentioned device or the like.

The reaction temperature in the reaction step is not particularly limited insofar as the above requirements are satisfied.

For example, the atmosphere temperature is preferably 250°C or less, more preferably 200°C or less, and further preferably 180°C or less.

If the atmosphere temperature during the heating is higher than 250 °C, thermal decomposition occurs as described above, or the discoloration of fibers is accelerated. On the other hand, if the reaction temperature is lower than 100°C, the reaction time tends to increase.

Therefore, in view of workability, the reaction temperature (specifically, the atmosphere temperature) during the heating is adjusted to not less than 100°C and not more than 250°C, more preferably not less than 100°C and not more than 200°C, and further preferably not less than 100°C and not more than 180°C.

Reaction Time in Reaction Step

The heating time (i.e., the reaction time) when the heating method described above is employed as the reaction step is not particularly limited insofar as sulfo groups can be appropriately introduced into the cellulose fiber as described above.

For example, the reaction time in the reaction step is adjusted to 1 minute or more if the reaction temperature is adjusted within the above range. More specifically, the heating time is preferably 5 minutes or more, more preferably 10 minutes or more, and further preferably 20 minutes or more.

If the reaction time is less than 1 minute, it is assumed that the reaction to cause the substitution of the sulfo groups with respect to the hydroxyl groups of the cellulose fibers hardly proceeds. On the other hand, if the heating time is excessively long, improvement in the introduction amount of sulfo groups tends to be less significant.

More specifically, the reaction time in the reaction step may be set so that a reaction solution is brought into contact with the fiber raw material and a part of the hydroxyl groups of the cellulose fibers in the fiber raw material can be substituted with sulfo groups. By reacting the sulfonated pulp fibers prepared through this reaction step with the reaction solution described above, the sulfonated pulp fibers can maintain a high fiber length retention rate (%) before and after the reaction.

Therefore, when the heating method described above is employed as the reaction step, the reaction time is preferably, but not particularly limited to, not less than 5 minutes and not more than 300 minutes, and more preferably not less than 5 minutes and not more than 120 minutes in view of reaction time and operability.

Sulfonating Agent

The sulfonating agent in the reaction step is not particularly limited insofar as it is a compound having sulfo groups.

Examples thereof include sulfamic acid, sulfamic acid salts, and sulfuryl compounds with sulfonyl group having two oxygen atoms covalently bonded to sulfur. These compounds may be used alone or in combination of 2 or more as the sulfonating agent.

The sulfonating agent is not particularly limited insofar as it is a compound described above. However, it is preferable to use sulfamic acid in view of the handling property because sulfamic acid has a lower acidity than sulfuric acid or the like, ensures high sulfo group introduction efficiency, and is inexpensive and highly safe.

Fiber Raw Material

The fiber raw material used in the method for producing the sulfonated fine cellulose fibers is not particularly limited insofar as it contains cellulose, as described above.

The fiber raw material may be any material insofar as it is constituted of cellulose molecules. Examples of fiber raw materials include those commonly referred to as pulp or those containing cellulose isolated from ascidians, seaweed, and the like.

Examples of the pulp include, but not limited to, wood-based pulp (hereinafter simply referred to as wooden pulp), dissolving pulp, cotton-based pulp such as cotton linter, wheat straw, bagasse, paper mulberry, paper birch, hemp, kenaf, non-wood-based pulp such as fruits, and waste-paper-based pulp produced from waste newspapers, waste magazines, or waste cardboards. Wood pulp is easy to use as a fiber raw material in terms of easy availability.

There are various kinds of wooden pulp. However, there is no particular limitation of use. Examples thereof include paper-making pulps such as Needle Bleached Kraft Pulp (NBKP), Leaf Bleached Kraft Pulp (LBKP), thermomechanical pulp (TMP), and the like.

When the pulps described above are used as the fiber raw material, the pulps described above may be used solely or in a combination of 2 or more.

Drying Step

The fiber raw material may contain moisture when it is supplied to the reaction step as described above, and, as long as this condition of the fiber raw material is maintained, a drying step may be performed between the contact step and the reaction step.

This drying step functions as a pretreatment step before the reaction step, and performs drying so that the moisture content of the fiber raw material after the contact step is reduced.

The moisture content of the fiber raw material after the drying step is preferably adjusted to 1% or more. If the moisture content of the fiber raw material after the drying step is lower than 1%, that is, if the fiber raw material in an absolute dry state is supplied to the reaction step, since the hydrogen bonds between fibers of the fiber raw material strongly act in the absolute dry state, the sulfonation reaction in the reaction step may not properly proceed. Therefore, in the drying step, the moisture content of the fiber raw material after the drying step is desirably adjusted to 1% or more.

In addition, the following advantages are obtained by performing a drying step.

When a fiber raw material having a high moisture content is supplied to the reaction step, there is a concern that a part of sulfamic acid, urea, or the like is subjected to hydrolysis due to the heating reaction in the reaction step, which results in a decrease in reaction efficiency. Moreover, depending on the conditions, a reaction not desired for a fiber raw material to be subjected to fibrillation may occur. Therefore, a drying step to lower the moisture content of the fiber raw material may be performed before the reaction step in view of suppressing the possibility of damages and the like during the heating reaction.

By performing a drying step, the possibility of damages and the like may be suppressed, the reaction time of the esterification reaction can be reduced, and the operability can be improved in the subsequent reaction step. In other words, by performing the drying step, the moisture in the reaction solution adhered to the fiber raw material after the contact step can be properly removed when the fiber raw material is supplied to the reaction step, thereby increasing the concentration of sulfamic acid in the fiber raw material to be supplied to the reaction step. This provides an advantage that the esterification reaction between the hydroxyl groups and sulfamic acid in the fiber raw material can be more easily promoted in the reaction step.

The method of the drying step is not particularly limited insofar as the moisture content of the fiber raw material after the drying step is adjusted to be 1% or more, as described above. For example, the drying step may be performed using a dryer to adjust the moisture content of the fiber raw material after the drying step to be almost in equilibrium with the moisture content in the atmosphere, or to be not more than 15%, not more than 10%, or even as low as about 5%.

In this specification, the equilibrium state means a state in which the moisture in the atmosphere in the treatment facility and the moisture in the raw material do not visually come in and out. Specifically, the equilibrium state refers to a condition in which, after drying for a certain period of time (e.g., 2 hours), the amount of change between the two continuous measurements is within 1% of the weight at the start of the drying (provided that the second weight measurement is at least a half of the drying time required for the first measurement).

The drying temperature in the drying step is not particularly limited. It is however desirable to perform the drying at a temperature lower than the heating temperature in the heating reaction in the reaction step. If the atmosphere temperature during the heating exceeds 100°C, the sulfonating agent and the like may be decomposed. On the other hand, if the atmosphere temperature during the heating is less than 20°C, the drying time increases. Therefore, the drying temperature in the drying step is such that the atmosphere temperature during the drying is preferably 100°C or less, more preferably not less than 20°C and not more than 100°C, further preferably not less than 50 °C and not more than 100 °C . Therefore, the atmosphere temperature during the drying in the drying step is preferably not more than 100°C. From the viewpoint of operability, the atmosphere temperature is preferably adjusted to not less than 20°C.

Washing Step

After the reaction step in the chemical treatment step S2, a washing step for washing the sulfonated pulp fibers in which sulfo groups are introduced may be performed.

The surface of the sulfonated pulp fiber after the introduction of sulfo groups is acidic by the influence of the sulfonating agent. In addition, an unreacted reaction solution is also present. Therefore, by performing a washing step that reliably terminates the reaction and removes the excess reaction solution to ensure a neutral state, the handling property can be improved.

The washing step is not particularly limited insofar as the sulfonated pulp fiber after the introduction of sulfo groups can be substantially neutralized. For example, a method of washing the sulfonated pulp fibers obtained after the introduction of sulfo groups with pure water or the like until the sulfonated pulp fibers become neutral can be employed. Further, neutralization washing using alkali or the like may also be performed. In the neutralization washing, examples of the alkali compounds contained in the alkali solution include inorganic alkali compounds, organic alkali compounds and the like. Examples of inorganic alkali compounds include hydroxides, carbonates, phosphates, and the like of alkali metals. Examples of organic alkali compounds include ammonia, aliphatic amines, aromatic amines, aliphatic ammonium, aromatic ammonium, heterocyclic compounds, and hydroxides of heterocyclic compounds.

Fibrillation Treatment Step S3

The fibrillation treatment step S3 is a step of fibrillating the sulfonated pulp fibers obtained in the chemical treatment step S2 into fine fibers having a predetermined size (for example, nano-level size).

The processing device (fibrillation device) used in the fibrillation treatment step S3 is not particularly limited insofar as it has the above-described function.

Examples of processing devices include, but not limited to, a low-pressure homogenizer, a high-pressure homogenizer, a grinder (stone mill type pulverizer), a ball mill, a cutter mill, a jet mill, a short-screw extruder, a twin-screw extruder, an ultrasonic stirrer, a household mixer, and the like. Among these, it is desirable to use a high-pressure homogenizer, which is capable of uniformly applying a force to the material and is also capable of excellent homogenization; however, the processing device is not limited to high-pressure homogenizers.

When a high-pressure homogenizer is used in the fibrillation treatment step S3, the sulfonated pulp fibers obtained by the above-described production method are supplied in a state of being dispersed in a water-soluble solvent, such as water. Hereinafter, the solution in which the sulfonated pulp fibers are dispersed is referred to as a slurry.

The solid concentration of the sulfonated pulp fibers in the slurry is not particularly limited. For example, a solution obtained by adjusting the solid concentration of the sulfonated pulp fibers in the slurry to 0.1% by mass to 20% by mass may be supplied to a processing device, such as a high-pressure homogenizer.

For example, when a slurry in which the solid concentration of the sulfonated pulp fibers is adjusted to 0.5% by mass is supplied to a processing device, such as a high-pressure homogenizer, a dispersion liquid in which the sulfonated fine cellulose fibers of the same solid concentration are dispersed in a water-soluble solvent can be obtained. That is, in this case, a dispersion liquid in which the solid concentration of the sulfonated fine cellulose fibers is adjusted to 0.5% by mass can be obtained.

When a high-pressure homogenizer is used as a processing device in the fibrillation treatment step S3, the fibrillation pressure and the number of times of fibrillation are not particularly limited, insofar as the sulfonated fine cellulose fibers with the desired viscosity characteristics (viscosity, TI value) can be appropriately prepared.

Adjustment by Fibrillation Pressure

For example, for the adjustment by the fibrillation pressure of a high-pressure homogenizer, the fibrillation pressure is preferably adjusted to be not more than 100 MPa, and more preferably not more than 60 MPa, when the solid concentration of the sulfonated pulp fibers in the slurry is 0.5 % by mass.

When the sulfonated pulp fibers are fibrillated with a high fibrillation force at a fibrillation pressure higher than 100 MPa, the number of unit fiber linkages tends to decrease and the fiber length of the sulfonated fine cellulose fibers is likely to decrease. This is presumably due to the fact that, according to the method for producing the sulfonated fine cellulose fibers, the sulfonated pulp fibers to be subjected to the fibrillation treatment step S3 have been depolymerized by the polymerization degree lowering treatment step S1.

Therefore, the fiber length, i.e., the number of unit fiber linkages of the sulfonated fine cellulose fibers obtained by using a fibrillation force at a pressure higher than 100 MPa tends to decrease. The decrease in the number of unit fiber linkages results in a decrease in the fiber length of the sulfonated fine cellulose fibers; as a result, the viscosity of the dispersion liquid thereof also tends to decrease.

Therefore, when a high-pressure homogenizer is used as the processing device (fibrillation device) in the fibrillation treatment step S3 and adjusts the viscosity of the sulfonated fine cellulose fibers using the fibrillation pressure thereof, the fibrillation pressure is preferably adjusted to not more than 100 MPa, more preferably not more than 60 MPa.

Further, since the viscosity of the sulfonated fine cellulose fibers tends to be correlated with the fibrillation pressure, the fibrillation pressure may be appropriately adjusted while being lowered when the sulfonated fine cellulose fibers with high viscosity are prepared. For example, the fibrillation pressure may be adjusted to be not more than 30 MPa or not more than 15 MPa. More specifically, the viscosity characteristics of the sulfonated fine cellulose fibers can be adjusted by adjusting the fibrillation pressure.

Adjustment by Number of Times of Fibrillation

Further, for example, for the adjustment by the number of times of fibrillation (number of passes) while keeping a constant fibrillation pressure of the high-pressure homogenizer, the adjustment is preferably made so that the number of times of fibrillation is 30 passes or less when the solid concentration of the sulfonated pulp fibers in the slurry is 0.5 % by mass. Further, in view of the operability or the like, the number of passes are preferably 25 passes or less, more preferably 20 passes or less, even more preferably 15 passes or less, and further more preferably 10 passes or less.

When the number of times of fibrillation is more than 30 passes, the number of unit fiber linkages tends to decrease, and the fiber length of the sulfonated fine cellulose fibers is likely to decrease. This is due to the same reason as in the adjustment by the fibrillation pressure described above.

Therefore, when a high-pressure homogenizer is used as the processing device (fibrillation device) in the fibrillation treatment step S3 so as to adjust the viscosity of the sulfonated fine cellulose fibers using the number of times of fibrillation (number of passes), the number of times of fibrillation is preferably adjusted within the above range.

Further, since the viscosity of the sulfonated fine cellulose fibers tends to be correlated with the number of times of fibrillation (number of passes), the sulfonated fine cellulose fibers with high viscosity may be prepared by decreasing the number of times of fibrillation (number of passes), and the sulfonated fine cellulose fibers with a low viscosity may be prepared by appropriately adjusting the number of times of fibrillation (number of passes) to increase. More specifically, the viscosity characteristics of the sulfonated fine cellulose fibers can be adjusted by adjusting the number of times of fibrillation (number of passes).

On the other hand, in view of productivity, a smaller number of times of fibrillation (number of passes) is preferable because the fewer the number of passes, the fewer operations are required, the higher the processing volume, the better the workability, and the lower the cost. In this viewpoint, the number of times of fibrillation (number of passes) is preferably 20 passes or less, more preferably 15 passes or less, even more preferably 10 passes or less, and further more preferably 5 passes or less.

Adjustment by Fibrillation Pressure and Number of Times of Fibrillation

The sulfonated fine cellulose fibers may be prepared to exhibit the desired viscosity characteristics (viscosity, TI value) by adjusting the fibrillation intensity based on the fibrillation pressure and the number of times of fibrillation.

For example, the fibrillation intensity can be adjusted by increasing the number of times of fibrillation (number of passes) while fixing the fibrillation pressure at 30 MPa or less.

The fibrillation intensity may also be defined as the fibrillation energy (J) imparted to the sulfonated pulp fibers during the fibrillation treatment of the sulfonated pulp fibers.

For example, the flow quantity at each fibrillation pressure is calculated based on the water flowing in the pipe of the fibrillation device used for the fibrillation treatment and the fibrillation pressure during the fibrillation treatment.

Then, the flow speed of the water is calculated from the flow quantity. Then, the kinetic energy (J) for each fibrillation treatment can be calculated based on the flow quantity and the fibrillation pressure at each fibrillation treatment. The calculation example in this method of expressing the fibrillation intensity in the form of fibrillation energy (J) is specifically described in the Examples below.

For example, to prepare sulfonated fine cellulose fibers that exhibit transparency (e.g., with a haze value of 20% or less), the fibers are prepared so that the fibrillation energy (J) is 10J or more. The fibrillation energy (J) is preferably 20J or more, more preferably 40J or more, and further preferably 50J or more (see FIG. 8). On the other hand, in view of productivity, it is sufficient that the fibrillation energy (J) is not more than 2,000J, preferably not more than 1,000J, more preferably not more than 500J, further preferably not more than 250J, and even more preferably not more than 100J.

Further, for example, in the preparation of sulfonated fine cellulose fibers that exhibit viscosity and transparency when made into a dispersion liquid, the lower limit of the fibrillation energy (J) is the same as the above value, and the upper limit may be not more than 500J, preferably not more than 100J.

Further, for example, in the preparation of sulfonated fine cellulose fibers that exhibit high viscosity and low TI value, and exhibit transparency when made into a dispersion liquid, the lower limit of the fibrillation energy (J) is the same as the above value, and the upper limit may be not more than 200J, preferably not more than 150J, and more preferably not more than 100J (see CNF containing the first maximum point in the Example of FIG. 6, FIG. 8, Table 1, Tables 5 to 9).

On the other hand, in the preparation of sulfonated fine cellulose fibers that exhibit transparency, high viscosity, and a high TI value when made into a dispersion liquid, the lower limit of the fibrillation energy (J) is the same as the above value, and the upper limit may be not less than 150J, preferably not less than 200J (see CNF containing the second maximum point in the Example of FIG. 6, FIG. 8, Table 1, Tables 5 to 9).

The relationship between the fibrillation treatment and the viscosity of the sulfonated fine cellulose fibers can be summarized as follows.

Heretofore, when a high-pressure homogenizer is used as a device (fibrillation device) for the fibrillation treatment, there has been a problem that the processing capacity greatly decreases as the fibrillation pressure of the high-pressure homogenizer increases. For example, when a versatile device that performs a fibrillation treatment at a fibrillation pressure of 100 MPa or more is used to produce fibers having the desired size, the processing amount per hour is several 100 liters; that is, the production efficiency is very low. In contrast, when the fibrillation is performed at a fibrillation pressure of several 10 MPa, the processing amount becomes several 1,000 liters per hour, which is significantly higher than the above case. However, since the fibrillation pressure is set to a low level in this case, there is a problem that the fibers cannot be fibrillated to the desired size.

On the other hand, the method for producing sulfonated fine cellulose fibers according to the present embodiment is capable of appropriately producing sulfonated fine cellulose fibers that can exhibit the desired viscosity characteristics (viscosity and TI value) even when the fibrillation pressure and the number of times of fibrillation (number of passes) in the fibrillation treatment step S3 are within the above range, as described above.

This is presumably due to the fact that, as described above, the sulfonated pulp fibers to be subjected to the fibrillation treatment step S3 have been depolymerized by the polymerization degree lowering treatment step S1. Therefore, it is assumed that the hydrogen bonds between the fibers of the sulfonated pulp fibers are weak, and the fibers more easily come apart.

Therefore, with this production method, as described above, smooth fibrillation (defiberization) can be performed. As a result, the time required for the fibrillation treatment step can be further reduced, thus improving the productivity of sulfonated fine cellulose fibers having the functions described above. For example, although the conventional technologies require a high fibrillation pressure and a large number of times of fibrillation during the fibrillation treatment, the method for producing sulfonated fine cellulose fibers according to the present embodiment allows the fibrillation treatment to be done with a lower fibrillation pressure and a smaller number of times of fibrillation.

Therefore, with this production method, it is possible to easily produce sulfonated fine cellulose fibers capable of exhibiting excellent viscosity characteristics as described above even at a low fibrillation pressure that cannot be assumed in the conventional methods. Moreover, since the number of times of fibrillation can be reduced, it is possible to improve the efficiency of the operation during the treatment. As a result, for example, as shown in Tables 5 and 6, sulfonated fine cellulose fibers exhibiting the desired viscosity characteristics can be produced with a smaller energy without decreasing productivity.

In addition, by adjusting the conditions in the fibrillation treatment in the fibrillation treatment step (e.g., the fibrillation pressure and/or the number of times of fibrillation (number of passes)), the viscosity characteristics, such as viscosity or TI value, of the sulfonated fine cellulose fibers can be controlled.

Therefore, by using the method for producing sulfonated fine cellulose fibers according to the present embodiment, it is possible to easily, efficiently, and appropriately produce sulfonated fine cellulose fibers that can exhibit excellent viscosity characteristics.

Examples

It was confirmed that the sulfonated fine cellulose fibers of the present invention can be produced by using the method for producing sulfonated fine cellulose fibers of the present invention. Further, it was also confirmed that the prepared sulfonated fine cellulose fibers of the present invention had predetermined characteristics.

Experiment 1

In Experiment 1, Needle Bleached Kraft Pulp (NBKP) (average fiber length = 2.6mm) produced by Marusumi Paper Co. Ltd. was used as a fiber raw material. Hereinbelow, NBKP used in the experiment is simply referred to as a pulp.
The pulp was washed with a large amount of pure water, the water was removed using a 200-mesh sieve, then the solid concentration was measured, and the pulp was subjected to the experiment without being dried.

Polymerization Degree Lowering Treatment Step

A treatment to reduce the polymerization degree of pulp having been washed with pure water was performed.
Sodium hydroxide (concentration = about 48%, produced by Sumitomo Chemical Co., Ltd.) was used as the reagent in the polymerization degree lowering treatment step. The reagents were adjusted at a concentration of 1 mol/L, and used as the treatment solutions for the polymerization degree lowering treatment.
Pulp was added to the prepared aqueous sodium hydroxide solution. The solid concentration of the pulp was adjusted to 10% relative to 1 mol of the aqueous sodium hydroxide solution, and the mixture was allowed to stand overnight at room temperature, thereby yielding pulp with a reduced polymerization degree.
For example, 100 g (dry weight) of pulp was added to 900 g of the reagent, and after the reagent and the pulp were evenly mixed, the mixture was allowed to stand at room temperature and atmospheric pressure for 24 hours to perform a polymerization degree lowering treatment.
The polymerization degree lowering treatment corresponds to the contact step of the polymerization degree lowering treatment step in the method for producing sulfonated fine cellulose fibers according to the present embodiment, and the reagent used corresponds to the polymerization degree lowering agent.

Washing Step after Polymerization Degree Lowering Treatment Step

The pulp with a reduced polymerization degree resulting from the polymerization degree lowering treatment step was neutralized with 0.5 M sulfuric acid, and it was confirmed that the pH was neutral. The pulp was then washed with a large amount of pure water and subjected to a chemical treatment step.

Chemical Treatment Step

The supplied pulp with a reduced polymerization degree was added to the reaction solution prepared as follows, immersed in a chemical liquid, and made into a slurry.
The step of adding the pulp with a reduced polymerization degree to the reaction solution to form a slurry corresponds to the contact step in the chemical treatment step of the method for producing sulfonated fine cellulose fibers of the present embodiment.

Preparation of Reaction Solution

The sulfonating agent and urea and/or a derivative thereof were prepared to have the following concentrations.
In the experiment, sulfamic acid (having a purity of 98.5%, manufactured by Fuso Chemical Co., Ltd.) was used as the sulfonating agent. As urea or a derivative thereof, a urea solution (having a purity of 99%, manufactured by Wako Pure Chemical Industries, Ltd., Model No.: special grade reagent) was used.
They were mixed at a mixing ratio (a concentration ratio (g/L)) of 2:1(1:0.5), thereby preparing an aqueous solution.
The sulfamic acid and the urea were mixel as follows. Ratio of sulfamic acid/urea ((g/L)/(g/L))=200/100
An example of the method for preparation of the reaction solution is shown below.
100 ml of water was added to a container. Then, 20 g of sulfamic acid and 10 g of urea were added to the container to prepare a reaction solution having a ratio of sulfamic acid/urea ((g/L)/(g/L)) of 200/100 (1:0.5). More specifically, urea was added in an amount of 50 parts by weight with respect to 100 parts by weight of sulfamic acid.

Contact Method for Reaction Solution and Pulp with Reduced Polymerization Degree

The pulp (dry weight) with a reduced polymerization degree was added to the prepared reaction solution. The slurry was adjusted so that the ratio of the pulp (dry weight) with a decreased polymerization degree to the chemicals in the reaction solution was: pulp:chemicals = 1:1.5.

In the experiment, in the case of a reaction solution having a sulfamic acid/urea ratio ((g/L)/(g/L)) of 200/100 (1:0.5), about 6.5 g of the reaction solution was added to 1 g (dry weight) of the pulp with a reduced polymerization degree so that the pulp was impregnated with the chemicals.

The slurry prepared by adding the pulp with a reduced polymerization degree to the reaction solution was kneaded with hands for 30 minutes, and the pulp with a reduced polymerization degree was uniformly impregnated with the reaction solution. The pulp with a reduced polymerization degree impregnated with the reaction solution was placed in a dryer (Model No.:VTR 115, manufactured by Isuzu Seisakusho). The temperature of the constant-temperature bath of the dryer was set to 50°C. The pulp with a reduced polymerization degree was dried until the moisture content was in an equilibrium state. Then, the pulp with a reduced polymerization degree having been dried prior to the heating reaction was supplied to the subsequent heating reaction step.

The moisture content of the pulp to be supplied to the heating reaction step, with a reduced polymerization degree and in contact with the reaction solution, was calculated using the following equation.

The "equilibrium state of moisture content" was evaluated as follows.

First, drying was performed for 1 hour in the dryer with a constant-temperature bath set at 50°C. Then, the equilibrium state was determined based on a condition in which the amount of change in weight between the two continuous measurements was within 1% of the weight at the start of the drying (however, the second weight measurement was at least half of the drying time required for the first measurement).

$$\text{Moisture content (\%)} = 100 \cdot (\text{weight (g) of solids content of the pulp/weight}$$
$$\text{(g) of the pulp at the time of moisture content measurement after the contact}$$
$$\text{with the reaction solution)} \times 100$$

The weight (g) of solids content of the pulp refers to the dry weight of the pulp to be measured, which, in this experiment, corresponds to 2 g of dry pulp used in the experiment. The weight of the dry pulp was measured after drying at 105°C for 2 hours using the dryer mentioned above.

In this experiment, the weight of the pulp after the drying using a dryer before being supplied to the heating reaction corresponds to the weight (g) of pulp at the time of moisture content measurement after the contact with the reaction solution.

In the experiment, the moisture content of the pulp after the drying was a few percent to about 10% (drying temperature = 50°C). More specifically, in this experiment, the drying step was performed in a manner such that the moisture content of the dried pulp did not fall to 1% or less (i.e., absolute dry state).

The step of drying the pulp with a reduced polymerization degree impregnated with the reaction solution corresponds to the drying step in the chemical treatment step of the method for producing sulfonated fine cellulose fibers of the present embodiment.

Heating Reaction

Then, the dry pulp obtained by drying the pulp with a reduced polymerization degree impregnated with the reaction solution was supplied to the subsequent heating reaction step to perform a heating reaction.

In the heating reaction, a dryer (Model No.:VTR 115, manufactured by Isuzu Seisakusho) was used.

The reaction conditions in the heating reaction were as follows. Temperature of constant-temperature bath: 120 °C. Heating time: 25 minutes.

This heating reaction corresponds to the reaction step in the chemical treatment step of the method for producing sulfonated fine cellulose fibers of the present embodiment.

This temperature as a reaction condition of the heating reaction corresponds to the reaction temperature in the reaction step in the chemical treatment step of the method for producing sulfonated fine cellulose fibers of the present embodiment.

This heating time as a reaction condition of the heating reaction corresponds to the reaction time in the reaction step in the chemical treatment step of the method for producing sulfonated fine cellulose fibers of the present embodiment.

After the heating reaction, the reacted pulp was neutralized using sodium hydrogencarbonate, and it was confirmed that the pH was neutral. Thereafter, the neutralized pulp was washed with a large amount of pure water to prepare sulfamic acid/urea-treated pulp.

The step of washing the reacted pulp with pure water until it becomes neutral corresponds to the washing step in the chemical treatment step of the method for producing sulfonated fine cellulose fibers of the present embodiment.

The reacted pulp fibers constituting the prepared sulfamic acid/urea-treated pulp correspond to the sulfonated pulp fibers of the present embodiment.

Fibrillation Treatment Step

Subsequently, the solid concentration of the prepared sulfamic acid/urea-treated pulp was adjusted to 0.5% by mass, and a fibrillation (nanosizing) treatment was performed using a high-pressure homogenizer (Model No. N2000-2C-045, manufactured by KOS21 Co.,Ltd.), thereby preparing nanocellulose fibers.

The treatment conditions of the high-pressure homogenizer were as follows.

First, preliminary fibrillation was carried out using a high-pressure homogenizer. The preliminary fibrillation was performed at a fibrillation pressure of 10 MPa with 2 times of fibrillation (2 passes).

Then, the slurry resulting from the preliminary fibrillation was supplied to a high-pressure homogenizer to be subjected to a fibrillation treatment at a fibrillation pressure of 15 MPa, thereby preparing nanocellulose fibers.

In addition, each nanocellulose fiber sample was prepared according to the number of times of fibrillation (number of passes) of the high-pressure homogenizer (Sample A; 3 passes, Sample B; 5 passes, Sample C; 7 passes, Sample D; 9 passes, Sample E; 11 passes, Sample F; 15 passes).

The fibrillation operation using the high-pressure homogenizer corresponds to the fibrillation treatment step in the method for producing sulfonated fine cellulose fibers of the present embodiment. The prepared nanocellulose fibers corresponds to the sulfonated fine cellulose fibers of the present embodiment.

Measurement of Sulfer Introduction Amount by Electrical Conductivity Measurement

The sulfur introduction amount attributable to sulfo group was measured by titration with an aqueous sodium hydroxide solution after the prepared nanocellulose fibers were treated with an ion exchange resin.

In the treatment with an ion-exchange resin, a strongly acidic ion-exchange resin (Amberjet 1024 (conditioned), manufactured by Organo Corporation) at a volume ratio of 1/10 was added to 100 mL of a slurry containing 0.2% by mass of nanocellulose fibers, and the mixture was subjected to a shaking treatment for 1 hour. Thereafter, the mixture was poured into a 200 pm-opening mesh, thereby separating the resin from the slurry.

In the titration using an aqueous sodium hydroxide solution, while adding 0. 5N aqueous sodium hydroxide solution in an amount of 10 to 50 pL each time to the nanocellulose fiber-containing slurry having been through the ion exchange resin treatment, changes in electrical conductivity values were measured. Then, a curve was obtained by plotting the electrical conductivity on the vertical axis and plotting the amount of sodium hydroxide titrated on the horizontal axis, and the inflection point was confirmed from the obtained curve.

The amount of sodium hydroxide titrated at this inflection point corresponds to the amount of sulfo groups. Therefore, by dividing the amount of sodium hydroxide at the inflection point by the amount of solids content of the nanocellulose fibers used in the measurement, the amount of the sulfo groups, i.e., the introduction amount of sulfur attributable to the sulfo groups in the nanocellulose fibers were measured.

Observation of Fiber Form and Measurement of Fiber Width Using SPM

The nanocellulose fibers obtained after the high-pressure homogenizer treatment were adjusted to have a solid concentration suitable for observation, i.e., a solid concentration of 0.0005 to 0.001% by mass, using pure water. Thereafter, the mixture was spin-coated onto a silica substrate coated with PEI (polyethyleneimine).

The nanocellulose fibers spin-coated on the silica substrate were observed using a scanning probe microscope (manufactured by Shimadzu Corporation, Model No.: SPM-9700).

The fiber width and the fiber length were measured by randomly selecting 20 fibers in the observation image.

Measurement of Haze Value and Measurement of Total Light Transmittance

The measurement of haze value and the measurement of total light transmittance were performed using a measurement solution, which was prepared by adjusting the solid concentration of nanocellulose fibers to 0.5 to 1.0% by mass with pure water.

When the slurry is prepared by adjusting the solid concentration of the sulfamic acid/urea-treated pulp (the solid concentration of treated pulp) in the slurry supplied to the high-pressure homogenizer to 0.5% by mass in the fibrillation treatment step, the solid concentration of the nanocellulose fibers in the dispersion liquid obtained after the fibrillation treatment step is also 0.5% by mass. Therefore, this dispersion liquid was not adjusted and used directly for the measurement with the solid concentration of the nanocellulose fiber of 0.5% by mass.

A predetermined amount of portion was separated from the prepared measurement solution to prepare a CNF slurry for measurement.

An integrating sphere (ISN-470, manufactured by JASCO Corporation) was attached to a spectrophotometer (Model No.: 0V-570 manufactured by JASCO Corporation) to measure the haze value and the total light transmittance as follows according to JIS K 7105.

A quartz cell containing pure water was used as a blank measurement value, and the light transmittance of a nanocellulose dispersion liquid of 0.5% by mass was measured. The measurement wavelength range was 380 to 780 nm.

The total light transmittance (%) and the haze value (%) were calculated from the numerical values obtained by the spectrophotometer using the bundled calculation software.

The measurement solution supplied to the spectrophotometer corresponds to the dispersion liquid in the method for producing a sulfonated fine cellulose fiber of the present embodiment.

Measurement of Polymerization Degree

The limiting viscosity (intrinsic viscosity) of the nanocellulose fibers was measured in accordance with JIS P 8215 (1998).

The JIS-standard limiting viscosity measurement has method A and method B. This experiment used "method A - Determination of limiting viscosity number of cellulose in dilute solutions". The measurement method was changed such that the pulp as the raw material and the nanocellulose fibers were subjected to the measurement by being fractioned so that each fraction has a solids content of 0.1 g. The average value of the results of three measurements was regarded as the measured value.

Then, the average polymerization degree (DP) of the nanocellulose fiber was calculated from each intrinsic viscosity ($\eta$) according to the following equation. Since the average polymerization degree is an average polymerization degree measured by a viscosity method, it may be referred to as "viscosity average polymerization degree".

$$DP = 1.75 \times [\eta]$$

Viscosity Measurement

The sample having been hermetically closed and allowed to stand at room temperature for 24 hours was used for the viscosity measurement. After the standing, 100 g of the sample was separated and placed in a 110 mL tall beaker, and the viscosity was measured using a Brookfield viscometer (LVDV-I Prime, manufactured by Eko Instruments Co., Ltd.).

The conditions in the viscosity measurement were as follows.

The rotation speed was 6 rpm, the measurement temperature was 25°C, the measurement time was 3 minutes, No. 64 spindle was used, and single-point data was used.

TI Value Measurement

The thixotropy index (TI value) was measured as follows.

The TI value was calculated by performing measurement using the Brookfield viscometer described above at a rotation speed of 6 rpm and 60 rpm, and determining the respective viscosities according to the following formulas. The other conditions were as described above.

$$\text{TI value} = (\text{viscosity at a rotation speed of 6 rpm})/(\text{viscosity at a rotation speed of 60 rpm})$$

Results of Experiment

The sulfur introduction amount attributable to sulfo groups in the nanocellulose fibers (Sample A; 3 passes, Sample B; 5 passes, Sample C; 7 passes, Sample D; 9 passes, Sample E; 11 passes, Sample F; 15 passes) was 1.0 to 1.5 mmol/g, with an average of 1.3 mmol/g.

The average polymerization degree of the sulfamic acid/urea-treated pulp fiber before being fibrillated was 404. The average polymerization degree of each nanocellulose fiber after the fibrillation treatment was 300 to 314, with an average of 308.

The average fiber width of each nanocellulose fiber was 30 nm or less in all cases.

The total light transmittance (%), haze value (%), viscosity (mPa·s), TI value, and average polymerization degree of each nanocellulose fiber are shown in the table below.

Table 1

|  | SAMPLE A; 3 PASSES | SAMPLE B; 5 PASSES | SAMPLE C; 7 PASSES | SAMPLE D; 9 PASSES | SAMPLE E; 11 PASSES | SAMPLE F; 15 PASSES |
|---|---|---|---|---|---|---|
| TOTAL LIGHT TRANSMITTANCE (%) | 97.8 | 98.6 | 98.9 | 98.9 | 98.1 | 99.1 |
| HAZE VALUE(%) | 6.5 | 3.5 | 2.4 | 1.8 | 1.8 | 0.8 |
| VISCOSITY (mPa·s) | 13897 | 17396 | 13197 | 10198 | 8998 | 7198 |
| TI VALUE | 5.6 | 8.1 | 8.1 | 7.4 | 8.1 | 7.6 |
| AVERAGE POLYMERIZATION DEGREE | 314 | 307 | 314 | 305 | 308 | 300 |

FIG. 2 shows the relationship between the number of times of fibrillation, the average polymerization degree, and the viscosity (mPa·s).

As shown in FIG. 2, it was confirmed that the viscosity can be controlled by controlling the number of times of fibrillation. The average polymerization degrees for the respective times of fibrillation slightly varied, but all of them were close to the levelling-off degree of polymerization of cellulose.

The experiment results revealed that the pulp having a reduced polymerization degree as a result of the polymerization degree lowering treatment was in a depolymerized state. Then, since the pulp in this state was chemically treated, it is inferred that the hydrogen bonds between the fibers of the sulfamic acid/urea-treated pulp were presumably weakened by the chemical treatment, and the fibers more easily came apart.

This presumably made it possible to prepare nanocellulose fibers with the desired viscosity and transparency even with a low fibrillation pressure and a small number of times of fibrillation (number of passes). In other words, the polymerization degree lowering treatment shortened the cellulose molecular chain and increased the fiber breakage. And it is inferred that the above phenomenon is caused by weakening the hydrogen bonds due to the reduction of crystalline regions in which fibers are properly aligned.

Further, if the number of times of fibrillation (number of passes) is changed while keeping the same fibrillation pressure, the viscosity tends to decrease when the number of times of fibrillation (number of passes) is more than 5 even though the polymerization degree of each sample of nanocellulose fiber stays substantially the same level. Therefore, it was inferred that the average polymerization degree of each nanocellulose fiber represents the average polymerization degree of the further finer cellulose fibers (corresponding to the unit fibers in this embodiment) constituting the nanocellulose fiber.

That is, according to the experiment result showing that the viscosity varied even though the average polymerization degree was almost the same for each nanocellulose fiber, the adjacent fine cellulose fibers (unit fibers) constituting the nanocellulose fiber are disconnected at each time of fibrillation (passes). This presumably resulted in the decrease in viscosity with the increase in the number of times of fibrillation (number of passes).

The above results confirmed that it is possible to appropriately and efficiently produce the sulfonated fine cellulose fibers with the desired excellent viscosity by using the method for producing sulfonated fine cellulose fibers of the present invention. For example, it was confirmed that the viscosity of the sulfonated fine cellulose fibers can be controlled by adjusting the number of times of fibrillation.

It was also confirmed that the obtained sulfonated fine cellulose fibers were in a state in which a plurality of unit fibers having an average polymerization degree near the levelling-off degree of polymerization of cellulose are connected together.

Experiment 2

Viscosity and Fiber Form

Nanocellulose fibers (hereinafter referred to as CNF) with different degrees of polymerization were prepared by the polymerization degree lowering treatment so as to confirm the influence of the polymerization degree on the viscosity and fiber form. In other words, in view of the relationship between the polymerization degree and the fiber form and viscosity, it was confirmed that CNF having high viscosity can be prepared even with a low polymerization degree.
Sample B (viscosity: 17396 mPa·s) of Experiment 1 was used as Sample 1.
Sample G (viscosity: 3,000 mPa·s) prepared by further fibrillating Sample F with a high strength was used as Sample 2. Sample G was made by subjecting Sample F (15 passes) to the fibrillation treatment five more times at 100 MPa using a similar fibrillation device.

Preparation of Comparative Sample 1

Sulfonated pulp was prepared without the polymerization degree lowering treatment to be used as Comparative Sample 1. The sample was made in the same manner as in Example 1, except that the polymerization degree lowering treatment was not performed, and the following conditions were adopted.
The sulfamic acid and the urea were mixed at a concentration ratio (g/L) of 2:5 (1:2.5). Specifically, 100 mL of water was added to a container, and 20 g of sulfamic acid and 50 g of urea were added to the container to prepare a reaction solution having a ratio of sulfamic acid/urea ((g/L)/(g/L)) of 200/500 (1:2.5). More specifically, urea was added in an amount of 50 parts by weight with respect to 100 parts by weight of sulfamic acid.
A slurry was prepared from the reaction solution thus prepared so that the ratio of chemicals in the reaction solution to each pulp (dry weight) was such that pulp:chemicals = 1:2.5.
In the experiment, in the case of a reaction solution having a sulfamic acid/urea ratio ((g/L)/(g/L)) of 200/500 (1:2.5), about 3.6 g of the reaction solution was added to 1 g (absolute dry weight) of the pulp so that the pulp was impregnated with the chemicals.
The fibrillation treatment was performed twice at a fibrillation pressure of 10 MPa, once at a fibrillation pressure of 50 MPa, and five times at a fibrillation pressure of 60 MPa using a NanoVater (L-ES008-D10, manufactured by Yoshida Kikai Co., Ltd.). In the following, a pressure with MPa means the fibrillation pressure of the fibrillation treatment device used in the fibrillation treatment.
After the comparative fibers were hermetically closed and allowed to stand at room temperature for 24 hours and then a portion of 50 g was placed in a 50 mL polypropylene Falcon tube (Corning), the viscosity was measured using a Brookfield viscometer (model number; RV-DV2T, manufactured by Eko Instruments Co., Ltd.).
The conditions in the viscosity measurement were as follows.
The rotation speed was 6 rpm, the measurement temperature was 25°C, the measurement time was 3 minutes, and RV-06 spindle was used.

Results of Experiment 2

In the prepared Comparative Sample 1, the sulfur introduction amount attributable to sulfo groups in the sulfonated fine cellulose fiber was 1.0 mmol/g.
It has previously been thought that, when the polymerization degree is low, the resulting CNF has a short fiber length, and does not ensure high viscosity. Therefore, in order to obtain CNF with high viscosity such as gels, the previously-known technologies performed a chemical treatment before the fibrillation treatment so as to prevent a decrease in polymerization degree. Such treatment made it possible to obtain CNF having transparency and high viscosity.
In view of such common technical knowledge, ensuring a high polymerization degree is considered necessary to prepare CNF having transparency and high viscosity.
On the other hand, in the present invention, in view of the relationship between the polymerization degree and the fiber form and viscosity, consideration as to whether CNF having high viscosity can be prepared even with a low polymerization degree was made.
For the sample, the pulp was prepared by varying the polymerization degree depending on whether or not the polymerization degree lowering treatment was performed. Each of the prepared pulps was subjected to the fibrillation treatment, and the polymerization degree, the fiber form, and the viscosity were examined for each fibrillation state.
Table 2 shows the characteristics of the samples and the results of various measurements.

Table 2

|  | POLYMERIZ ATION DEGREE | VISCOSITY (mPa · s) |
|---|---|---|
| SAMPLE 1 | 307 | 17396 |
| COMPARATIVE SAMPLE 1 | 420 | 15000 |

The definition of CNF in the present invention is such that the preparation of CNF (i.e., fibrillation) was completed when the haze fell to or below 20%.

The results of Experiment 1 shown in Table 1 and the results of this experiment shown in Table 2 reveal that CNF with a polymerization degree near 300 after the fibrillation was prepared by performing a polymerization degree lowering treatment (alkaline treatment) before the sulfonation step. According to the results of changes in polymerization degree and viscosity for each fibrillation state of these pulps, it was revealed that the change in polymerization degree was little for each fibrillation state, and the viscosity reached a maximum value and then decreased.

Table 1 of Experiment 1 and Table 2 of this experiment reveal that all of the maximum values of the CNF viscosity exceed 15,000 mPa·s, which is similar to that of CNF having a polymerization degree of 400 in Comparative Sample 1, regardless of whether the polymerization degree lowering treatment was performed. These results suggested that the viscosity does not completely depend on the polymerization degree.

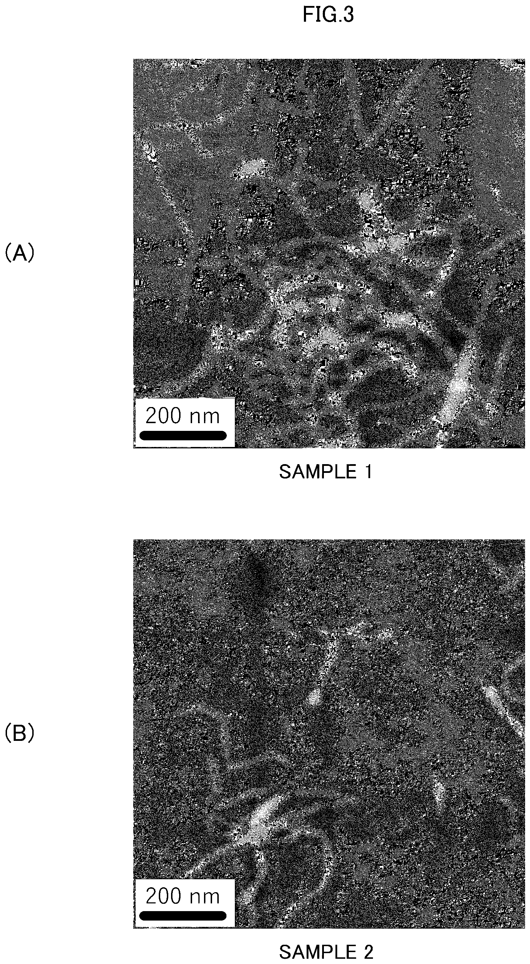

FIG. 3 shows the observation results of fiber forms of CNFs having substantially the same polymerization degree and various viscosities.

The photograph in FIG. 3(A) shows Sample 1, and the photograph in FIG. 3(B) shows Sample 2.

For Sample 1 with a viscosity of 10,000 mPa•s or higher, entangled fibers with a width of several nm and a fiber length of 1 $\mu$m to 2 $\mu$m were frequently observed (FIG. 3(A)). On the other hand, for Sample 2 with a viscosity of about 3,000 mPa•s, the fibers were less entangled with each other and many of them were a few nm wide and about 500 nm long (FIG. 3(B)).

The results confirmed that the viscosity does not depend on the polymerization degree; rather, it is more important to prepare CNF with a greater fiber length and more entanglement of fibers in the fibrillation step.

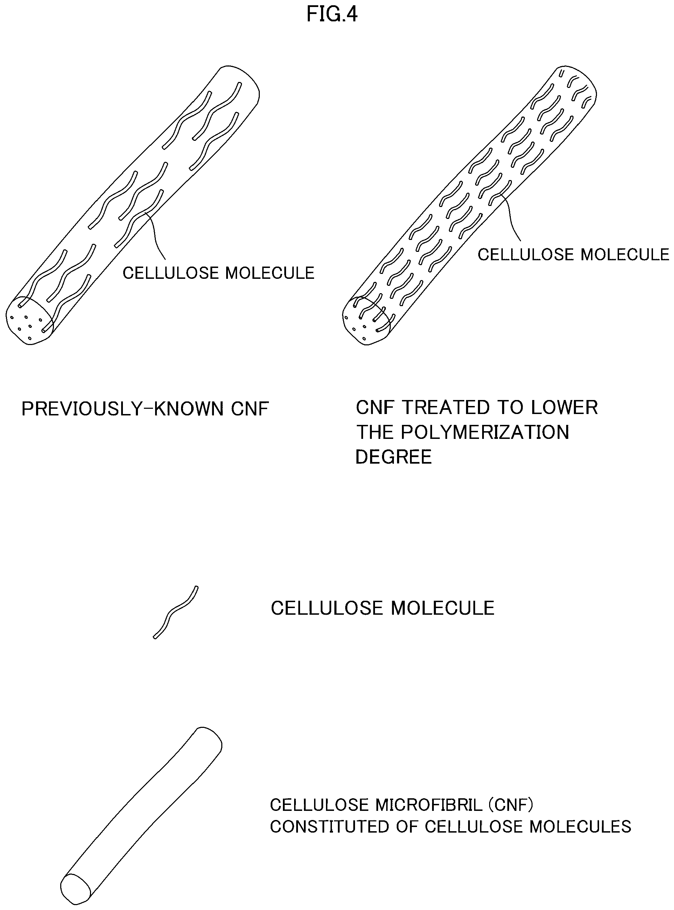

From these results, it was confirmed that the production method of the present invention makes it possible to prepare, as shown in the image of FIG. 4, CNF with a width of several nm and a length of several $\mu$m constituted of cellulose molecules having been treated to lower the polymerization degree (corresponding to the unit fibers of the present embodiment; the left figure of FIG. 4) as the sulfonated fine cellulose fibers of the present invention.

In other words, according to the results shown above, it is presumably possible to prepare CNF constituted of cellulose molecules with a different length even when the viscosity is the same (CNF length is the same). According to such a structure, it was confirmed that the CNF can exhibit high viscosity despite the low polymerization degree.

Experiment 3

Color Test

In Experiment 3, the influence of the polymerization degree lowering treatment on the color was confirmed.

In this experiment, the sulfonated fine cellulose fibers (Sample 3) were prepared in the same manner as in Experiment 1, except for the conditions described below.

In the fibrillation treatment, a high-pressure homogenizer (Model No. N2000-2C-045, manufactured by KOS21 Co.,Ltd.) was used.

The solid concentration of the sulfonated pulp was adjusted to 1.0%.

The fibrillation treatment was performed twice at a fibrillation pressure of 10 MPa, once at a fibrillation pressure of 50 MPa, and three times at a fibrillation pressure of 60 MPa.

The color of Sample 3 was measured using a spectral haze meter.

Color Measurement

The color was measured using a spectral haze meter (model number; SH-7000, manufactured by Nippon Denshoku Industries Co., Ltd.) as follows.

An optional glass cell (part number: 2277, square cell, optical path length 10 mm $\times$ 40 (width) $\times$ 55 (height)) of the above spectral haze meter was used to measure the light transmittance of the pulp slurry to be measured. For the blank measurement, the value obtained by placing pure water in the glass cell of the above spectral haze meter was used as the blank measurement value.

The measurement conditions were such that the light source was D65, the viewing angle was 2°, the measurement wavelength range was 380 to 780 nm.

The measurement was performed according to JIS K 7105.

The L*, a*, b* values were obtained by a control unit (model number: CU-II, Ver. 2.00.02) of the spectral haze meter.

Measurement of Polymerization Degree

The measurement of polymerization degree was performed in the same manner as in Experiment 1.

Sample 4

In this experiment, Sample 4 was further prepared and analyzed in the same manner as for Sample 3.

Sample 4 was prepared in the same manner as that for Sample 3, except that the sulfamic acid/urea ratio ((g/L)/(g/L)) was 200/200 in the preparation conditions of the sulfonated pulp in Experiment 1. In Sample 4, the introduction amount of sulfur attributable to the sulfo groups was 1.5 mmol/g.

Comparative Sample 2

Comparative Sample 2 was prepared in the same manner as that for Sample 3, except that the polymerization degree lowering treatment was not performed. In Comparative Sample 2, the introduction amount of sulfur attributable to the sulfo groups was 1.3 mmol/g.

Results of Experiment 3

Table 3 shows the results of Experiment 3.

Table 3

| | COLOR | | | TRANSPARENCY | |
|---|---|---|---|---|---|
| | L* VALUE | A* VALUE | B* VALUE | TOTAL LIGHT TRANSMITTANCE (%) | HAZE VALUE (%) |
| SAMPLE 3 | 99.1 | 0.00 | 0.85 | 97.6 | 7.5 |
| SAMPLE 4 | 99.34 | -0.05 | 0.71 | 98.3 | 8.1 |
| COMPARATIVE SAMPLE 2 | 98.88 | 0.00 | 0.95 | 97.1 | 6.1 |

In addition to being highly transparent, there may be situations where CNF is required to be colorless. Therefore, the influence of the polymerization degree lowering treatment on the color of CNF was examined.

As a result, the polymerization degree of Samples 3 and 4 were both near 300, while the polymerization degree of Comparative Sample 2 was about 400.

Table 3 shows the influence on the color of CNF depending on whether or not the polymerization degree lowering treatment was performed.

In both Samples 3 and 4 in which the polymerization degree lowering treatment was performed, the L* value was high, compared with Comparative Sample 2. In addition, the fact that a* and b* values were close to zero indicates that the samples were colorless. This is presumably because the color components were removed during the sulfonation treatment of the pulp having been subjected to the polymerization degree lowering treatment.

Experiment 4

Biodegradability Test

CNF produced by mechanically treating cellulose extracted from a wooden material is generally regarded biodegradable. However, the biodegradability of chemically treated CNF is often uncertain. Biodegradability is a property to decompose of substances by the action of bacteria and enzymes in nature. CNF are degraded unless there is a problem in the substrate specificity of the degrading enzyme. However, for example, there are problems in the biodegradability of chemically modified cellulose. For example, although cellulose acetate, in which cellulose has been esterified, is biodegradable, the degradation speed is slower than that of cellulose.

Considering the environmental burden in using CNF, it is desirable to increase the biodegradability of CNF as much as possible.

Therefore, in this experiment, an examination as to whether or not the sulfonated fine cellulose fibers are biodegradable was performed. Then, the influence of the polymerization degree on the biodegradability was confirmed based on the results.

Sample 5

Sulfonated pulp was prepared by the same method as in Experiment 1, and the obtained sulfonated pulp was subjected to a fibrillation treatment to prepare sulfonated fine cellulose fibers (Sample 5).

The fibrillation treatment was performed using a high-pressure homogenizer, NanoVater (L-ES008-D10, manufactured by Yoshida Kikai Co., Ltd.).

The solid concentration of the sulfonated pulp subjected to the fibrillation treatment was 0.5%.

The fibrillation treatment was performed twice at a fibrillation pressure of 10 MPa, once at a fibrillation pressure of 50 MPa, and then three times at a fibrillation pressure of 60 MPa. The prepared Sample 5 was subjected to the following enzymatic treatment.

Measurement of Degradability of Sample 5 by Enzymatic Treatment

20 g of Sample 5 adjusted to 0.5% and 30 g of 0.1 M acetate buffer solution were placed in a 50 mL polypropylene Falcon tube (Corning), and 10 mg of cellulolytic enzyme (Aspergillus niger-derived cellulase, product code: 07550-74, Nacalai Tesque, Inc.) was added. The mixture was then placed in a constant-temperature bath set at 50°C and reacted for 24 hours.

At each reaction time, the solution was appropriately collected, and filtered through a 0.1 $\mu$m filter, followed by glucose analysis by HPLC.

A high performance liquid chromatography (10A series, manufactured by Shimadzu Corporation) was used for the glucose analysis. A glucose analysis column (Aminex glucose analysis column HPX-87P) manufactured by Bio-Rad was used as a column. A differential refractometer was used for the detection. Ultrapure water was used as the mobile phase.

The detection time was confirmed using glucose as the reference substance.

The peak obtained after the enzymatic treatment of Sample 5 and the HPLC detection peak of glucose were at substantially the same time. Therefore, it was assumed that glucose was obtained from Sample 5 by the enzymatic treatment. For quantitative determination, a calibration curve was obtained using glucose of known concentration, and calculation was performed.

Comparative Sample 3

Comparative Sample 3 was prepared using the same production method as that of Comparative Sample 1 in Experiment 2, except that the fibrillation treatment was performed with the following conditions.

The solid concentration of the sulfonated pulp subjected to the fibrillation treatment was 0.5%.

The fibrillation treatment was performed twice at a fibrillation pressure of 10 MPa, once at a fibrillation pressure of 50 MPa, and then five times at a fibrillation pressure of 60 MPa.

The prepared Comparative Sample 3 was subjected to enzymatic treatment in the same manner as that for Sample 5, and glucose analysis was performed also in the same manner.

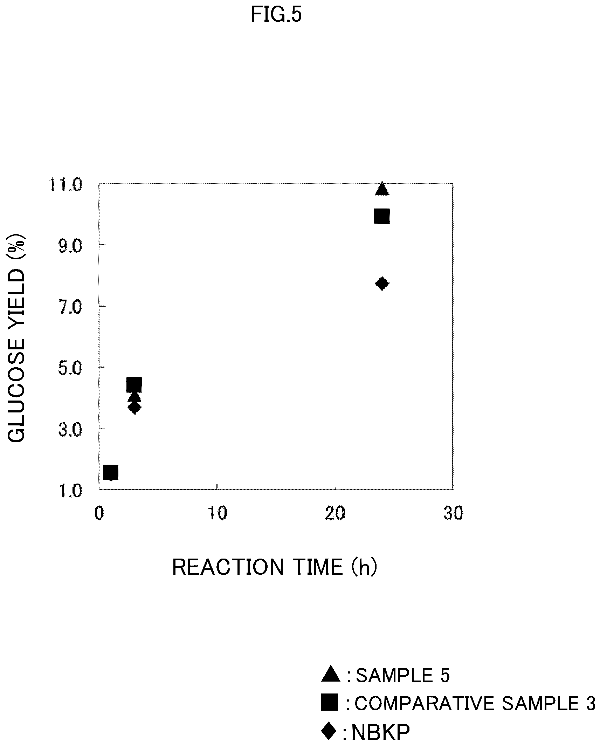

NBKP manufactured by Marusumi Paper Co. Ltd. (NBKP in FIG. 5, hereinafter simply referred to as NBKP) was used as the blank sample.

Results of Experiment 4

Cellulase having a cellulose degradation capability was used to confirm biodegradability.

The polymerization degree of Sample 5 was around 300, the polymerization degree of Comparative Sample 3 was around 400, and the polymerization degree of NBKP was around 900.

FIG. 5 shows the results of the enzymatic degradation rate.

CNF was converted to glucose over time, regardless of the polymerization degree.

The experiment results confirmed that the sulfonated cellulose was degraded by cellulase, i.e., it is biodegradable.

Comparing the degradation rate at each polymerization degree, the yield of glucose of Sample 5 (triangle in FIG. 5) was 1.6%→4.1%→10.9%. The yield of Comparative Sample 3 (square in FIG. 5) was 1.6%→4.4%→9.9%, and the yield of NBKP (rhombus in FIG. 5) was 1.5%→3.7%→7.7%.

The results confirmed that CNF with a lower polymerization degree had superior biodegradability than CNF with a higher polymerization degree.

In the experiment where the enzyme amount was increased tenfold, the yield of glucose of Sample 5 exceeded 80% after 24 hours.

Experiment 5

Dispersibility Test

Since it is generally believed that the structure and chemical characteristics of CNF are derived from the viscosity and TI value, it is expected that the use of CNF will be further expanded if such viscosity characteristics can be controlled. In this experiment, it was confirmed that CNF with predetermined viscosity characteristics can be controlled by performing the polymerization degree lowering treatment.

The results of Experiment 2 revealed that, with the increase in the fibrillation intensity, the sulfonated pulp first reached the maximum viscosity and then decreased.

Therefore, this experiment attempted to confirm changes in fiber dispersion state (change in viscosity) at a more delicate level by setting a lower fibrillation intensity.

In this experiment, the influence of the polymerization degree lowering treatment on the dispersibility was determined from the viscosity characteristics for each fibrillation state.

Sample 6

In this experiment, the sulfonated fine cellulose fibers (Sample 6) were prepared in the same manner as in Experiment 1, except for the conditions described below.

In the conditions of Sample B in Experiment 1, the polymerization degree lowering treatment step was adjusted so that the solid concentration of pulp was 10 % by mass in a 25 % by mass sulfuric acid solution. The sample was then neutralized with a 0.5M aqueous sodium hydroxide solution. A slurry was adjusted so that the sulfamic acid/urea ratio ((g/L)/(g/L)) was 200/200 and the ratio of chemicals in the reaction solution to each pulp (dry weight) was such that pulp:chemicals = 1:10, with a reaction temperature of 140°C and a reaction time of 30 minutes.

The fibrillation treatment was performed using a high-pressure homogenizer (NanoVater, model No.: L-ES008-D10, manufactured by Yoshida Kikai Co., Ltd.).

The fibrillation was performed by setting the solid concentration of the pulp to 0.5% with the fibrillation intensity of 1-7 scales. The fibrillation intensity was set as follows.

Fibrillation intensity 1: once at 10 MPa,
Fibrillation intensity 2: twice at 10 MPa,
Fibrillation intensity 3: three times at 10 MPa,
Fibrillation intensity 4: four times at 10 MPa,
Fibrillation intensity 5: three times at 10 MPa and twice at 30 MPa,
Fibrillation intensity 6: three times at 10 MPa, twice at 30 MPa, and twice at 60 MPa, and
Fibrillation intensity 7: seven times at 100 MPa.

The fibrillation intensity was set so that it increases in accordance with an increase in the fibrillation intensity value.

The measurement of viscosity was performed in the same manner as that for Comparative Sample 1 in Experiment 2.

The measurement of polymerization degree was performed in the same manner as in Experiment 1. The CNF prepared with a fibrillation intensity of 6 was used.

The measurements of the total light transmittance and haze value were performed in the same manner as in Experiment 3, except that the solid concentration of CNF was 0.5%.

Comparative Sample 4

Comparative Sample 4 was prepared using the same production method as Comparative Sample 1 in Experiment 2, except that the fibrillation treatment was performed under the following conditions.

The fibrillation was performed by setting the solid concentration of the pulp to 0.5% with the fibrillation intensity of 1-9 scales.

The fibrillation intensity was set as follows.

Fibrillation intensity 1: 200 mL for 2 minutes, "high" setting of a mixer (model number: MX-X701, manufactured by Panasonic Corporation).

The fibrillation intensities 2-9 were set as follows, in addition to the treatment at fibrillation intensity 1.

Fibrillation intensity 2: once at 10 MPa,
Fibrillation intensity 3: three times at 10 MPa and twice at 30 MPa, Fibrillation intensity 4: three times at 10 MPa, twice at 30 MPa, and once at 60 MPa,
Fibrillation intensity 5: three times at 10 MPa, twice at 30 MPa, and twice at 60 MPa,
Fibrillation intensity 6: three times at 10 MPa, twice at 30 MPa, and four times at 60 MPa,
Fibrillation intensity 7: three times at 10 MPa, twice at 30 MPa, twice at 60 MPa, and twice at 100 MPa,
Fibrillation intensity 8: three times at 10 MPa, twice at 30 MPa, twice at 60 MPa, and five times at 100 MPa, and
Fibrillation intensity 9: three times at 10 MPa, twice at 30 MPa, twice at 60 MPa, and ten times at 100 MPa.

The fibrillation intensity was set so that it increases in accordance with an increase in the fibrillation intensity value. That is, Comparative Sample 4 was subjected to fibrillation at a fibrillation intensity higher than that for Sample 6.

The viscosity, transparency, and polymerization degree of Comparative Sample 4 obtained were measured in the same manner as that for Sample 6.

The sulfonated fine cellulose fibers obtained with a fibrillation intensity 5 were used for the measurement of polymerization degree.

Results of Experiment 5

Table 4 shows the relationship between the polymerization degree of unfibrillated sulfonated pulp and the polymerization degree of fibrillated sulfonated fine cellulose fibers (hereinafter simply referred to as CNF). In Sample 6, the introduction amount of sulfur attributable to the sulfo groups was 1.6 mmol/g.

Table 4

| | POLYMERIZATION DEGREE | |
| --- | --- | --- |
| | BEFORE FIBRILLATION | AFTER FIBRILLATION |
| SAMPLE 6 | 420 | 330 |
| COMPARATIVE SAMPLE 4 | 860 | 420 |

As shown in Table 4, the polymerization degree of the sulfonated pulp having been subjected to the polymerization degree lowering treatment was 420, and the polymerization degree of the sulfonated pulp having not been subjected to the polymerization degree lowering treatment was 860.

The CNF exhibiting thickening properties (high viscosity) and a high TI value means that the viscosity is developed due to the entanglement of fibers in a static state; and that, however, when a predetermined force is applied, the entanglement of the fibers collapses. It is considered that the thinner the fiber width and the longer the fiber length, the stronger the fiber entanglement that contributes to the viscosity. On the other hand, in order to prepare CNF that exhibits thickening properties (high viscosity) and a low TI value, it is necessary to establish a dispersion state in which, unlike CNF with a high TI value, the entanglement of fibers does not collapse even when a predetermined force is applied.

Therefore, in this experiment, the dispersion states of CNF with different polymerization degrees were determined based on the viscosities and TI values.

Table 5 shows changes in viscosity, TI value, and transparency (total light transmittance and haze value) at each fibrillation intensity. FIG. 6 shows a relationship between the setting of fibrillation intensity and the viscosity/TI value.

Table 5

| | FIBRILLATION ENERGY FOR EACH FIBRILLATION INTENSITY(J) | | | | | | |
|---|---|---|---|---|---|---|---|
| | FIBRILLATION INTENSITY 1 | FIBRILLATION INTENSITY 2 | FIBRILLATION INTENSITY 3 | FIBRILLATION INTENSITY 4 | FIBRILLATION INTENSITY 5 | FIBRILLATION INTENSITY 6 | FIBRILLATION INTENSITY 7 |
| | 14.7 | 29.4 | 44.1 | 58.8 | 132.2 | 308.5 | 1468.3 |
| | TOTAL LIGHT TRANSMITTANCE FOR EACH FIBRILLATION INTENSITY(%) | | | | | | |
| | FIBRILLATION INTENSITY 1 | FIBRILLATION INTENSITY 2 | FIBRILLATION INTENSITY 3 | FIBRILLATION INTENSITY 4 | FIBRILLATION INTENSITY 5 | FIBRILLATION INTENSITY 6 | FIBRILLATION INTENSITY 7 |
| | 96.1 | 98.0 | 98.8 | 98.0 | 99.5 | 99.6 | 99.6 |
| | HAZE VALUE FOR EACH FIBRILLATION INTENSITY(%) | | | | | | |
| SAMPLE 6 | FIBRILLATION INTENSITY 1 | FIBRILLATION INTENSITY 2 | FIBRILLATION INTENSITY 3 | FIBRILLATION INTENSITY 4 | FIBRILLATION INTENSITY 5 | FIBRILLATION INTENSITY 6 | FIBRILLATION INTENSITY 7 |
| | 43.4 | 12.0 | 5.7 | 3.5 | 2.0 | 1.8 | 0.7 |
| | VISCOSITY FOR EACH FIBRILLATION INTENSITY (mPa·s) | | | | | | |
| | FIBRILLATION INTENSITY 1 | FIBRILLATION INTENSITY 2 | FIBRILLATION INTENSITY 3 | FIBRILLATION INTENSITY 4 | FIBRILLATION INTENSITY 5 | FIBRILLATION INTENSITY 6 | FIBRILLATION INTENSITY 7 |
| | 9200 | 13750 | 9625 | 8250 | 9200 | 12170 | 3000 |
| | TI VALUE FOR EACH FIBRILLATION INTENSITY | | | | | | |
| | FIBRILLATION INTENSITY 1 | FIBRILLATION INTENSITY 2 | FIBRILLATION INTENSITY 3 | FIBRILLATION INTENSITY 4 | FIBRILLATION INTENSITY 5 | FIBRILLATION INTENSITY 6 | FIBRILLATION INTENSITY 7 |
| | 3.1 | 3.5 | 1.8 | 1.7 | 2.4 | 8.9 | 7.1 |

The haze of Sample 6, which was subjected to the polymerization degree lowering treatment, was 20% or less when fibrillated at a fibrillation intensity 2. This confirmed that CNF was properly obtained. The polymerization degree of fibrillated CNF was about 330. After the coarse fibers were no longer observed in the visual observation of the CNF dispersion, no significant change in the polymerization degree was observed.

As shown in FIG. 6(A), it was revealed that two maximum points of viscosity were observed as the viscosity behavior of Sample 6 in a fibrillation treatment performed at a fibrillation pressure of 10 MPa. The two maximum points of viscosity had similar values. As shown in Table 5 and FIG. 6(B), it was revealed that the range of TI value when the viscosity is at the maximum points was such that the TI value at the first maximum point is 1.5 to 4, and the TI value at the second maximum point was 4 or more. In other words, since different TI values were observed in the CNF having the same degree of viscosity, it was inferred that the fiber dispersion state was different between the CNF in the range including the first maximum point and the CNF in the range including the second maximum point.

Table 6 shows changes in viscosity, TI value, and transparency (total light transmittance and haze value) at each fibrillation intensity of Comparative Sample 4.

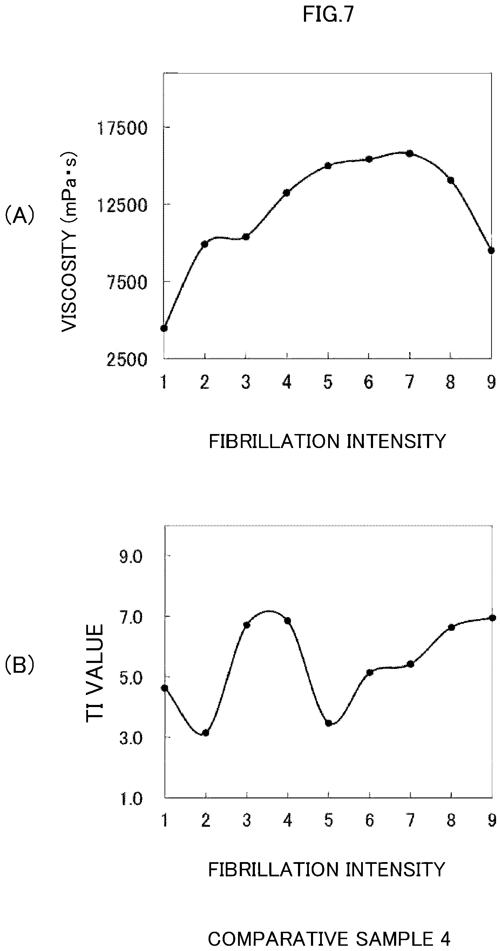

FIG. 7 shows a relationship between the setting of fibrillation intensity and the viscosity/TI value of Comparative Sample 4.

Table 6

| | FIBRILLATION ENERGY FOR EACH FIBRILLATION INTENSITY (J) | | | | | | | | |
|---|---|---|---|---|---|---|---|---|---|
| | FIBRILLATION INTENSITY 1 | FIBRILLATION INTENSITY 2 | FIBRILLATION INTENSITY 3 | FIBRILLATION INTENSITY 4 | FIBRILLATION INTENSITY 5 | FIBRILLATION INTENSITY 6 | FIBRILLATION INTENSITY 7 | FIBRILLATION INTENSITY 8 | FIBRILLATION INTENSITY 9 |
| | - | 14.7 | 132.2 | 220.3 | 308.5 | 484.7 | 602.1 | 1042.6 | 1776.7 |
| | TOTAL LIGHT TRANSMITTANCE FOR EACH FIBRILLATION INTENSITY(%) | | | | | | | | |
| | FIBRILLATION INTENSITY 1 | FIBRILLATION INTENSITY 2 | FIBRILLATION INTENSITY 3 | FIBRILLATION INTENSITY 4 | FIBRILLATION INTENSITY 5 | FIBRILLATION INTENSITY 6 | FIBRILLATION INTENSITY 7 | FIBRILLATION INTENSITY 8 | FIBRILLATION INTENSITY 9 |
| | 82.0 | 93.0 | 97.4 | 98.3 | 98.8 | 99.2 | 99.4 | 99.4 | 99.5 |
| | HAZE VALUE FOR EACH FIBRILLATION INTENSITY(%) | | | | | | | | |
| COMPARATIVE SAMPLE 4 | FIBRILLATION INTENSITY 1 | FIBRILLATION INTENSITY 2 | FIBRILLATION INTENSITY 3 | FIBRILLATION INTENSITY 4 | FIBRILLATION INTENSITY 5 | FIBRILLATION INTENSITY 6 | FIBRILLATION INTENSITY 7 | FIBRILLATION INTENSITY 8 | FIBRILLATION INTENSITY 9 |
| | 86.6 | 77.7 | 20.6 | 8.9 | 4.7 | 1.9 | 1.4 | 1.6 | 1.0 |
| | VISCOSITY FOR EACH FIBRILLATION INTENSITY(mPa·s) | | | | | | | | |
| | FIBRILLATION INTENSITY 1 | FIBRILLATION INTENSITY 2 | FIBRILLATION INTENSITY 3 | FIBRILLATION INTENSITY 4 | FIBRILLATION INTENSITY 5 | FIBRILLATION INTENSITY 6 | FIBRILLATION INTENSITY 7 | FIBRILLATION INTENSITY 8 | FIBRILLATION INTENSITY 9 |
| | 4500 | 9900 | 10400 | 13250 | 15000 | 15415 | 15790 | 14085 | 9500 |
| | TI VALUE FOR EACH FIBRILLATION INTENSITY | | | | | | | | |
| | FIBRILLATION INTENSITY 1 | FIBRILLATION INTENSITY 2 | FIBRILLATION INTENSITY 3 | FIBRILLATION INTENSITY 4 | FIBRILLATION INTENSITY 5 | FIBRILLATION INTENSITY 6 | FIBRILLATION INTENSITY 7 | FIBRILLATION INTENSITY 8 | FIBRILLATION INTENSITY 9 |
| | 4.6 | 3.2 | 6.7 | 6.8 | 3.5 | 5.1 | 5.4 | 6.6 | 7.0 |

In Comparative Sample 4, the polymerization degree of the pulp before fibrillation was high (800 or more), and CNF was not obtained with the same setting of fibrillation intensity as that for Sample 6. Therefore, for Comparative Sample 4, the fibrillation was performed at a setting of higher intensity.

As shown in FIG. 7, Comparative Sample 4, which used pulp with a high polymerization degree, had a haze of 20% or less at a setting of the fibrillation intensity of 3 (equivalent to the setting of fibrillation intensity 5 in Sample 6) and thereafter. It was thus confirmed that, for Comparative Sample 4, CNF was obtained at this setting of intensity for the first time. The obtained Comparative Sample 4 had a polymerization degree of about 400 and a TI value of about 3.5, which are higher than those of Sample 6.

The results shown above confirmed that this production method is capable of preparing CNF at a lower setting of fibrillation intensity than that in the previously-known production methods. In other words, it was confirmed that this production method is capable of producing transparent CNF with an energy lower than that in the previously-known production methods.

In addition, it was confirmed that the CNF can be prepared with a low range of fibrillation intensity had high viscosity and low TI value, and that CNF can be prepared with a high range of fibrillation intensity had high viscosity and high TI value. CNF with a high TI value presumably has a structure such that, when a force is applied to a dispersion of CNF, the entanglement of CNF easily comes apart. On the contrary, CNF with a low TI value presumably has a structure such that, when a force is applied to a dispersion of CNF, the entanglement of CNF does not easily come apart.

Therefore, it was confirmed that production this method is capable of preparing CNF with an energy lower than that in the previously-known production methods, while enabling the control to prepare CNF with different structures. Furthermore, it was also confirmed that the CNF obtained by this production method exhibited high transparency when dispersed at a predetermined concentration. It was also confirmed that this production method is capable of preparing CNF with high viscosity and a low or high TI value in a controlled manner even though the polymerization degree is low.

Experiment 6

Fibrillation Property

The influences of the polymerization degree lowering treatment on the fibrillation property of sulfonated pulp were examined.

In this experiment, the influences of the polymerization degree lowering treatment on the fibrillation property of sulfonated pulp was evaluated in view of the relationship between the fibrillation energy and the polymerization degree in the fibrillation treatment.

In this experiment, the fibrillation energy (J) required for the fibrillation treatment was determined based on the device used for the fibrillation treatment and the prepared Sample 6 (Sample 6 in Experiment 5).

Calculation of Fibrillation Energy (J)

First, the completion of the pulp fibrillation treatment was determined, as in other experiments, when the haze value of the CNF dispersion liquid (CNF slurry) obtained after the fibrillation treatment was 20% or less. This is because the haze value of the dispersion liquid of 20% or less indicates that the fiber width of the resulting CNF is approximately 20 nm or less, and that the transparency of the slurry can be visually confirmed.

Next, the following considers the energy applied to the pulp at each pressure of the high-pressure homogenizer used for the fibrillation.

The same nozzle of the fibrillation device was used in the experiment (manufactured by Yoshida Kikai Co., Ltd., nozzle model No.: cross type XT260, groove width = 160 μm), and water was used as the liquid. Therefore, at each pressure, the kinetic energy was calculated from the flow speed of the water flowing in the pipe, and the obtained kinetic energy was regarded as the fibrillation energy, and a comparison of the fibrillation state was performed based on the fibrillation energy.

Since this calculation method considers theoretical flow speed change due to the change in pressure of water flowing in a horizontal pipe when water is used as the solvent and the flow path or the like is constant, the influence of viscosity or pipe resistance was not taken into consideration.

The flow quantity and the flow speed of the liquid flowing through the pipe can be determined from the following equation.

$$Q = C \times A \times V \ (\text{Q: flow quantity, C: flow coefficient, V: flow speed, A: flow path area})$$

The flow speed can be calculated according to the following equation based on the Bernoulli's principle.

$$V = (2 \times P \div \rho)^{\wedge} 0.5 \ (\text{p: Differential Pressure}, \rho: \text{Fluid Density})$$

This equation reveals that the flow quantity is proportional to $(2 \times P \div \rho)^{\wedge} 0.5$ (i.e., proportional to the square root of pressure P) and inversely proportional to the square root of density ρ.

It can be assumed that, when the resistance is not considered, pressure P is proportional to the change in the pressure set in the fibrillation device (high-pressure homogenizer). In other words, when the pressure set in the device changes from 10 MPa to 30 MPa, the pressure is tripled in the flow speed V = $(2 \times P \div \rho)^{\wedge} 0.5$, based on Bernoulli's principle. Therefore, the flow speed V is increased by a factor of √3 (i.e., the flow speed is increased by a factor of 1.73) (provided that the fluid density and flow quantity coefficient are constant). Then, by calculating the energy of this fluid based on the kinetic energy formula (kinetic energy = $1/2mv^2$ ), the energy of the fluid can be determined as the energy given to the pulp in this experiment as the fibrillation force.

The following considers the flow speed and the kinetic energy at each pressure.

The maximum pressure set in the fibrillation device (high-pressure homogenizer) used in this experiment was 200 MPa, and the discharging flow quantity at that time was 7.8 mL/s. Since the flow rate is proportional to the square root of the pressure P, the flow quantity at 10 MPa is 7.8/√20 ≈ 1.74 cm³/s. Table 7 shows each pressure and the corresponding flow quantity.

Table 7

| PRESSURE (MPa) | FLOW QUANTITY (cm³/s) |
|---|---|
| 200 | 7.80 |
| 10 | 1.74 |
| 30 | 3.02 |
| 60 | 4.27 |
| 100 | 5.52 |

The nozzle groove width (nozzle diameter) of the fibrillation device used in the experiment was 160 μm. Assuming that the nozzle is a circular pipe, the flow path area is 0.00020096 cm². Then, the flow speed can be calculated according to the equation: flow speed (cm/s) = (flow quantity (cm³/s))/(flow path area (cm²)).

Table 8 shows the flow speed for each pressure.

Table 8

| PRESSURE (MPa) | FLOW SPEED (cm/s) |
|---|---|
| 200 | 38814 |
| 10 | 8679 |
| 30 | 15032 |
| 60 | 21259 |
| 100 | 27445 |

According to the values in Table 8, when the pressure is set to 10 MPa and the one-shot volume of the device is set to 3.9 mL, 3.9 g of water (density of water is 1 g/cm³ ) is running at 8679 cm/s in the pipe with a nozzle diameter of 160 μm. The kinetic energy at each pressure under these conditions is calculated according to the following equation. Table 9 shows the kinetic energy (J) at each pressure.

$$\text{Kinetic energy (J)} = 1/2mv^2 \ (\text{m: mass (kg)}, v: \text{speed (m/s)})$$

Table 9

| PRESSURE (MPa) | KINETIC ENERGY (J) |
|---|---|
| 200 | 293.7 |

(continued)

| PRESSURE (MPa) | KINETIC ENERGY (J) |
|---|---|
| 10 | 14.7 |
| 30 | 44.1 |
| 60 | 88.1 |
| 100 | 146.8 |

The kinetic energy (J) shown in Table 9 is the fibrillation energy (J) given to the pulp upon fibrillation of pulp.

Then, based on the relationship between the pressure and the kinetic energy (J) shown in Table 9, the fibrillation energy (J) at each setting of fibrillation intensity in this experiment is calculated. The influence of the number of treatments on each setting of fibrillation intensity was calculated by multiplying the value of fibrillation energy (J) for each time at each pressure by the number of treatments. FIG. 8 shows the calculation results.

FIG. 8 shows the relationship between the fibrillation energy required to prepare each CNF and the polymerization degree.

The graph in FIG. 8(B) is an enlarged view of the range surrounded by the dotted line in the graph in FIG. 8(A). The arrows in FIG. 8 indicate that the line shows a haze value of 20%, above which the pulp is not fibrillated, and below which the fibrillation is completed to thereby yield appropriate CNF.

In Sample 6 (square in FIG. 8), the haze value already fell below 20% at around fibrillation energy of 20J. On the other hand, Comparative Sample 4 (circle in FIG. 8) required fibrillation energy of 130J or more to decrease the haze value to 20% or less.

The above results confirmed that the energy for the fibrillation could be set much lower in Sample 6 than that for Comparative Sample 4, and that, therefore, Sample 6 had a superior fibrillation property compared with Comparative Sample 4.

Therefore, in the production method of the present invention, due to the polymerization degree lowering treatment, it becomes possible to yield the sulfonated fine cellulose fibers of the present invention (hereinafter simply referred to as CNF of the present invention) having a low TI value, which are difficult to prepare from pulp having a high polymerization degree. It was also confirmed that the CNF of the present invention, which exhibits such high transparency and high viscosity, can be prepared using very small energy compared with previously-known production methods.

In addition, the fibers of the CNF of the present invention produced by the production method of the present invention are supple due to the low polymerization degree, and can therefore make a variety of dispersion forms. In addition, very small energy is required for production.

Further, the CNF of the present invention exhibits viscosity characteristics that were not obtained by previously-known technologies. Specifically, the CNF of the present invention is those having high transparency, high viscosity, and low TI values, or those having high transparency, high viscosity, and a high TI value. The former CNF of the present invention has a fiber structure that maintains the entanglement between fibers even when external stress is applied, while the latter CNF of the present invention has a fiber structure in which the entanglement between fibers is easily collapsed by external stress.

Therefore, the CNF of the present invention obtained by the production method of the present invention can be used in various fields. For example, the CNF may be used for a drug delivery system, or, because of its capability to maintain the fiber form, high-viscosity products such as jellies, viscosity-controlling products for food, thickening agents for cosmetics, industrial products such as paints or operating fluids for hydraulic equipment, experimental devices such as culture media for cells, microorganisms, and viruses or crystal nucleator, soft materials such as contact lenses, and seismic isolation devices such as oil damping components or seismic isolators.

Further, the production method of the present invention is capable of adjusting the fibrillation energy (J) as described above, thereby controlling the TI value (i.e., fiber structure) of the obtained CNF of the present invention. Therefore, the CNF is expected to be usable as a viscosity control agent, such as grease.

Industrial Applicability

The sulfonated fine cellulose fibers and the method for producing sulfonated fine cellulose fibers according to the present invention can be suitably used for many usages in various fields, such as industrial fields, food fields, medical fields, and cosmetic fields, and can also be suitably used as a raw material of a composite material used in these fields.

Reference Numerals

S1: Polymerization Degree Lowering Treatment Step
S2: Chemical Treatment Step
S3: Fibrillation Treatment Step

**Claims**

1. A sulfonated fine cellulose fiber, which is a fine cellulose fiber obtained by fibrillating a cellulose fiber,

   a part of hydroxyl groups of the fine cellulose fiber being substituted with sulfo groups,
   an introduction amount of sulfur attributable to the sulfo groups being adjusted to be higher than 0.42 mmol/g,
   the fine cellulose fiber comprising a plurality of unit fibers and having an average fiber width of 30 nm or less, and
   a dispersion liquid in which the fine cellulose fiber is dispersed in a water-soluble solvent at a solid concentration
   of 0.5% by mass having a viscosity of 5,000 mPa·s or more at 25°C.

2. The sulfonated fine cellulose fiber according to claim 1, wherein a thixotropy index that is determined by measuring the dispersion liquid in which the fine cellulose fiber is dispersed in a water-soluble solvent at a solid concentration of 0.5% by mass using a Brookfield viscometer at rotation speeds of 6 rpm and 60 rpm at 25°C, calculating each viscosity, and determining each viscosity ratio (viscosity at a rotation speed of 6 rpm/viscosity at a rotation speed of 60 rpm) is 5.0 or more.

3. The sulfonated fine cellulose fiber according to claim 1, wherein a thixotropy index determined by measuring the dispersion liquid in which the fine cellulose fiber is dispersed in a water-soluble solvent at a solid concentration of 0.5% by mass using a Brookfield viscometer at rotation speeds of 6 rpm and 60 rpm at 25°C, calculating each viscosity, and determining each viscosity ratio (viscosity at a rotation speed of 6 rpm/viscosity at a rotation speed of 60 rpm) is less than 5.0.

4. The sulfonated fine cellulose fiber according to claim 1, 2 or 3, wherein the unit fibers have an average polymerization degree of 350 or less.

5. The sulfonated fine cellulose fiber according to claim 1, 2, 3 or 4, wherein the dispersion liquid in which the fine cellulose fiber is dispersed in a water-soluble solvent at a solid concentration of 0.5% by mass has a haze value of 10% or less.

6. The sulfonated fine cellulose fiber according to claim 1, 2, 3, 4 or 5, wherein the dispersion liquid in which the fine cellulose fiber is dispersed in a water-soluble solvent at a solid concentration of 0.5% by mass has a total light transmittance of 90% or more.

7. The sulfonated fine cellulose fiber according to any one of claims 1 to 6, wherein the L*a*b* color space, which is determined according to JIS Z 8781-4:2013, of the dispersion liquid in which the fine cellulose fiber is dispersed in a water-soluble solvent at a solid concentration of 0.5% by mass satisfies at least one of the following conditions:

   L* is 95% or more;
   a* is not less than -5 and not more than +5; and
   b* is not less than -5 and not more than +5.

8. A method for producing a sulfonated fine cellulose fiber, which is a method for producing the sulfonated fine cellulose fiber according to any one of claims 1 to 7 from pulp,

   the method performing a chemical treatment step of chemically treating the pulp and a fibrillation treatment step of fibrillating the pulp having been subjected to the chemical treatment step in this order,
   the method comprising a polymerization degree lowering treatment step of depolymerizing the pulp before the chemical treatment step.

9. The method for producing a sulfonated fine cellulose fiber according to claim 8, wherein, in the polymerization degree lowering treatment step, the pulp is brought into contact with an acid solution or an alkali solution, or is immersed in the acid solution or the alkali solution to prepare depolymerized pulp.

EP 4 029 983 A1

**10.** The method for producing a sulfonated fine cellulose fiber according to claim 8 or 9, wherein, in the fibrillation treatment step, the pulp having been subjected to the chemical treatment step is supplied to a fibrillation device and is fibrillated at a fibrillation pressure of 5 MPa to 60 MPa.

**11.** The method for producing a sulfonated fine cellulose fiber according to claim 10, wherein in the fibrillation treatment step, the pulp having been subjected to the chemical treatment step is repeatedly fibrillated by a fibrillation device, and the number of fibrillations is 30 passes or less.

**12.** The method for producing a sulfonated fine cellulose fiber according to claim 8, 9, 10, or 11, wherein, in the fibrillation treatment step, fibrillation energy of 1,000J or less is given to the pulp having been subjected to the chemical treatment step.

# FIG.1

```
┌─────────────────────────────────────┐
│                PULP                  │
└─────────────────────────────────────┘
                    │
                    ▼
┌─────────────────────────────────────┐
│  POLYMERIZATION DEGREE               │
│  LOWERING TREATMENT                  │
│  STEP(S1)                            │
└─────────────────────────────────────┘
                    │
                    ▼
┌─────────────────────────────────────┐
│  CHEMICAL TREATMENT STEP             │
│  (S2)                                │
└─────────────────────────────────────┘
                    │
                    ▼
┌─────────────────────────────────────┐
│  SULFONATED PULP FIBERS              │
└─────────────────────────────────────┘
                    │
                    ▼
┌─────────────────────────────────────┐
│  FIBRILLATION TREATMENT STEP         │
│  (S3)                                │
└─────────────────────────────────────┘
                    │
                    ▼
┌─────────────────────────────────────┐
│  SULFONATED FINE CELLULOSE           │
│  FIBERS                              │
└─────────────────────────────────────┘
```

FIG.2

NUMBER OF TIMES OF FIBRILLATION TREATMENTS

● : POLYMERIZATION DEGREE

■ : VISCOSITY

## FIG.3

(A)

SAMPLE 1

(B)

SAMPLE 2

# FIG.4

PREVIOUSLY-KNOWN CNF

CNF TREATED TO LOWER
THE POLYMERIZATION
DEGREE

CELLULOSE MOLECULE

CELLULOSE MICROFIBRIL (CNF)
CONSTITUTED OF CELLULOSE MOLECULES

FIG.5

▲ : SAMPLE 5
■ : COMPARATIVE SAMPLE 3
◆ : NBKP

FIG.6

(A)

(B)

SAMPLE 6

# FIG.7

(A)

FIBRILLATION INTENSITY

(B)

FIBRILLATION INTENSITY

COMPARATIVE SAMPLE 4

FIG.8

## INTERNATIONAL SEARCH REPORT

| | International application No. |
|---|---|
| | PCT/JP2020/034293 |

**A. CLASSIFICATION OF SUBJECT MATTER**
D06M 101/06(2006.01)n; A61Q 1/00(2006.01)i; D21H 11/20(2006.01)i; A61K 8/73(2006.01)i; C08B 5/14(2006.01)i; D06M 13/256(2006.01)i
FI: C08B5/14; A61Q1/00; A61K8/73; D21H11/20; D06M13/256; D06M101:06
According to International Patent Classification (IPC) or to both national classification and IPC

**B. FIELDS SEARCHED**

Minimum documentation searched (classification system followed by classification symbols)
D06M101/06; A61Q1/00; D21H11/20; A61K8/73; C08B5/14; D06M13/256

Documentation searched other than minimum documentation to the extent that such documents are included in the fields searched
| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| P, X | JP 2020-124680 A (NATIONAL UNIVERSITY CORPORATION UNIVERSITY OF FUKUI) 20 August 2020 (2020-08-20) production example 1, etc. | 1–12 |
| P, X | JP 2020-097812 A (MARUSUMI PAPER COMPANY LIMITED) 25 June 2020 (2020-06-25) paragraphs [0043]-[0062], fig. 3, etc. | 1–12 |
| P, X | JP 2020-097733 A (MARUSUMI PAPER COMPANY LIMITED) 25 June 2020 (2020-06-25) paragraphs [0041]-[0057], fig. 1-2, etc. | 1–12 |
| P, X | JP 2020-050739 A (DKS CO., LTD.) 02 April 2020 (2020-04-02) paragraphs [0032]-[0040], table 1, etc. | 1–12 |
| P, X | JP 2020-050738 A (DKS CO., LTD.) 02 April 2020 (2020-04-02) paragraphs [0033]-[0041], table 1, etc. | 1–12 |

☒ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 November 2020 (02.11.2020) | 17 November 2020 (17.11.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2020/034293 |

C (Continuation). DOCUMENTS CONSIDERED TO BE RELEVANT

| Category* | Citation of document, with indication, where appropriate, of the relevant passages | Relevant to claim No. |
| --- | --- | --- |
| P, X | JP 2020-050736 A (DKS CO., LTD.) 02 April 2020 (2020-04-02) paragraphs [0037]-[0045], table 1, etc. | 1-12 |
| P, X | WO 2019/208656 A1 (MARUSUMI PAPER COMPANY LIMITED) 31 October 2019 (2019-10-31) paragraphs [0045]-[0066], experiment examples, fig. 2, etc. | 1-12 |
| P, X | JP 6582111 B1 (MARUSUMI PAPER COMPANY LIMITED) 25 September 2019 (2019-09-25) paragraphs [0035]-[0052], experiment examples, fig. 4, etc. | 1-12 |
| P, X | JP 6582110 B1 (MARUSUMI PAPER COMPANY LIMITED) 25 September 2019 (2019-09-25) paragraphs [0032]-[0040], experiment examples, fig. 4, etc. | 1-12 |
| X | WO 2019/073810 A1 (DKS CO., LTD.) 18 April 2019 (2019-04-18) claim 3, table 1, paragraph [0044], etc. | 1-12 |
| Y | claim 3, table 1, paragraph [0044], etc. | 1-12 |
| X | WO 2019/003774 A1 (DKS CO., LTD.) 03 January 2019 (2019-01-03) claim 1, table 1, paragraph [0023], etc. | 1-12 |
| Y | claim 1, table 1, paragraph [0023], etc. | 1-12 |
| A | JP 2003-313201 A (WAKAMOTO PHARMACEUTICAL CO., LTD.) 06 November 2003 (2003-11-06) entire text | 1-12 |
| A | JP 52-013585 A (LION CORP.) 01 February 1977 (1977-02-01) entire text | 1-12 |

Form PCT/ISA/210 (continuation of second sheet) (January 2015)

INTERNATIONAL SEARCH REPORT
Information on patent family members

International application no.

PCT/JP2020/034293

| Patent Documents referred in the Report | Publication Date | Patent Family | Publication Date |
|---|---|---|---|
| JP 2020-124680 A | 20 Aug. 2020 | (Family: none) | |
| JP 2020-097812 A | 25 Jun. 2020 | (Family: none) | |
| JP 2020-097733 A | 25 Jun. 2020 | (Family: none) | |
| JP 2020-050739 A | 02 Apr. 2020 | (Family: none) | |
| JP 2020-050738 A | 02 Apr. 2020 | (Family: none) | |
| JP 2020-050736 A | 02 Apr. 2020 | (Family: none) | |
| WO 2019/208656 A1 | 31 Oct. 2019 | JP 2019-194299 A JP 2019-194300 A JP 2019-194389 A JP 2019-194390 A JP 6582110 B1 | |
| JP 6582111 B1 | 25 Sep. 2019 | JP 2019-194300 A JP 2019-194390 A JP 2019-194299 A JP 2019-194389 A WO 2019/208656 A1 | |
| JP 6582110 B1 | 25 Sep. 2019 | JP 2019-194299 A JP 2019-194389 A JP 2019-194300 A JP 2019-194390 A WO 2019/208656 A1 | |
| WO 2019/073810 A1 | 18 Apr. 2019 | JP 2019-70084 A | |
| WO 2019/003774 A1 | 03 Jan. 2019 | JP 2019-11411 A CN 110770257 A KR 10-2020-0023290 A | |
| JP 2003-313201 A | 06 Nov. 2003 | (Family: none) | |
| JP 52-013585 A | 01 Feb. 1977 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017170908 A

- JP 2019011411 A

**Non-patent literature cited in the description**

- *INDUSTRIAL AND ENGINEERING CHEMISTRY,* 1950, vol. 42 (3), 502-507